# EUROPEAN PATENT APPLICATION

(11) **EP 4 542 483 A1**
(43) Date of publication of application: **23.04.2025**
(21) Application number: 23823724.2
(22) Date of filing: 02.06.2023
(51) Int. Cl.: G06Q 50/22

(54) **NURSING-CARE ROBOT, METHOD FOR CONTROLLING NURSING-CARE ROBOT, AND INFORMATION PROCESSING DEVICE**

(30) Priority: 15.06.2022 JP 2022096284
(71) Applicant: Sony Group Corporation, Tokyo 108-0075 (JP)
(72) Inventor: SODEYAMA Yoshinao, Tokyo 108-0075 (JP); MIZUTANI Satoko, Tokyo 108-0075 (JP)
(74) Representative: D Young & Co LLP
(86) International application number: PCT/JP2023/020593
(87) International publication number: WO 2023/243431

(57) **Abstract**

The present technology relates to a nursing care robot, a method for controlling the nursing care robot, and an information processing device that allows for improved receptiveness of care recipients to various kinds of care provided by the nursing care robot.

The nursing care robot according to the present disclosure includes an action control unit configured to execute a cognitive approach that stepwise reduces a cognitive distance of a care recipient to content provided in an application for providing care to the care recipient and then to provide the content. For example, the present technology can be applied to a nursing care robot that provides care to a care recipient.

## Description

### [Technical Field]

The present technology relates to nursing care robots, methods for controlling a nursing care robot, and information processing devices, and particularly to a nursing care robot that performs various types of care for care recipients, a method for controlling such a nursing care robot, and an information processing device.

### [Background Art]

Various types of care such as music therapy have been carried out for people with dementia (see for example PTL 1).

For example, in care facilities such as nursing homes, there has been a demand for the development of nursing care robots that can provide various types of care to care recipients such as people with dementia.

### [Citation List]

### [Patent Literature]

[PTL 1]
JP 2020-14716A

### [Summary]

### [Technical Problem]

However, people with dementia may not readily accept music therapy if it is simply conducted by having a nursing care robot play music used in the music therapy. For example, people with dementia have difficulty in recognizing what music has started, why it has been played, whether they can sing at the moment, or when they should start singing.

The present technology has been developed in view of the foregoing circumstances and is directed to improving the receptiveness of a care recipient for example with dementia to various types of care including music therapy provided by a nursing care robot.

### [Solution to Problem]

A nursing care robot according to a first aspect of the present technology includes an action control unit configured to execute a cognitive approach that stepwise reduces the cognitive distance of a care recipient to content provided in an application for providing care to the care recipient and then to provide the content.

A method for controlling a nursing care robot configured to execute an application for providing care to a care recipient according to a second aspect of the present technology includes causing the nursing care robot to execute a cognitive approach that stepwise reduces the cognitive distance of the care recipient to content to be provided in the application and then to provide the content.

An information processing device according to a third aspect of the present technology includes an action control unit configured to cause a nursing care robot, which executes an application for providing care to a care recipient, to execute a cognitive approach that stepwise reduces the cognitive distance of the care recipient to content to be provided in the application and then to provide the content.

According to the first aspect, after executing a cognitive approach that stepwise reduces the cognitive distance of a care recipient to content provided in an application for providing care to the care recipient, the content is provided.

According to the second or third aspect, the nursing care robot, which executes an application for providing care to a care recipient, executes a cognitive approach that stepwise reduces the cognitive distance of the care recipient to content to be provided in the application, and then the content is provided.

### [Brief Description of Drawings]

[Fig. 1]

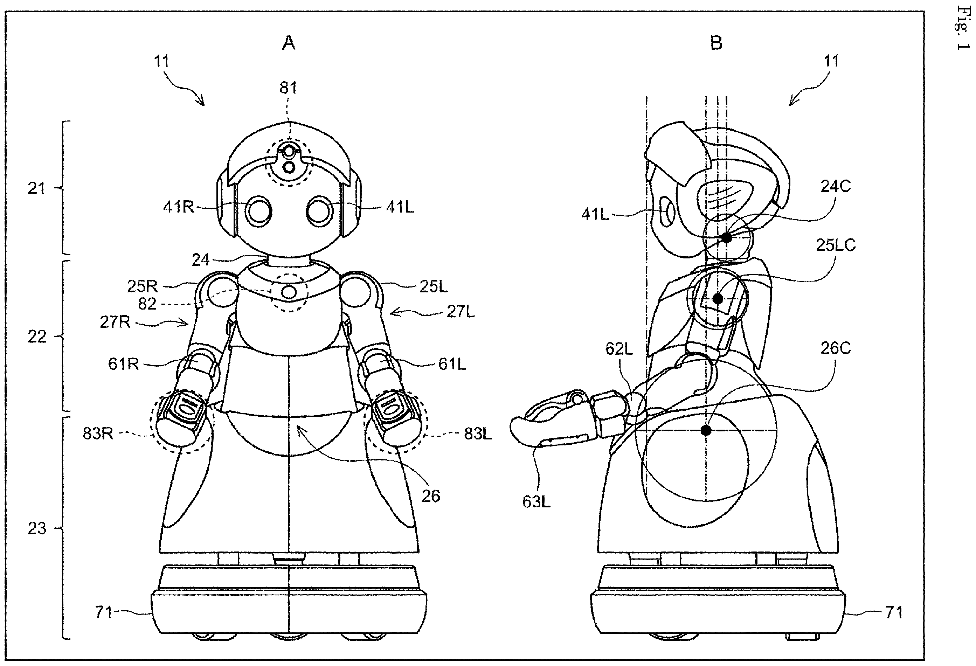

   Fig. 1 is a front view and a left side view of a nursing care robot to which the present technology is applied.
[Fig. 2]

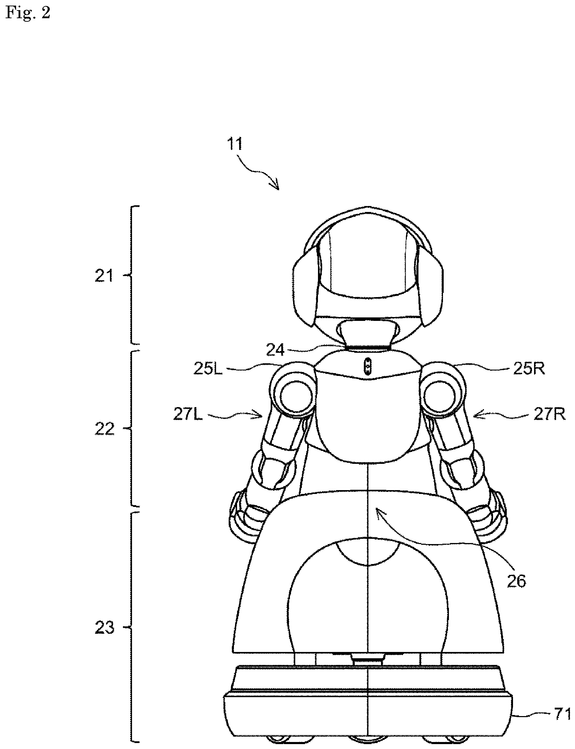

   Fig. 2 is a rear view of the nursing care robot.
[Fig. 3]

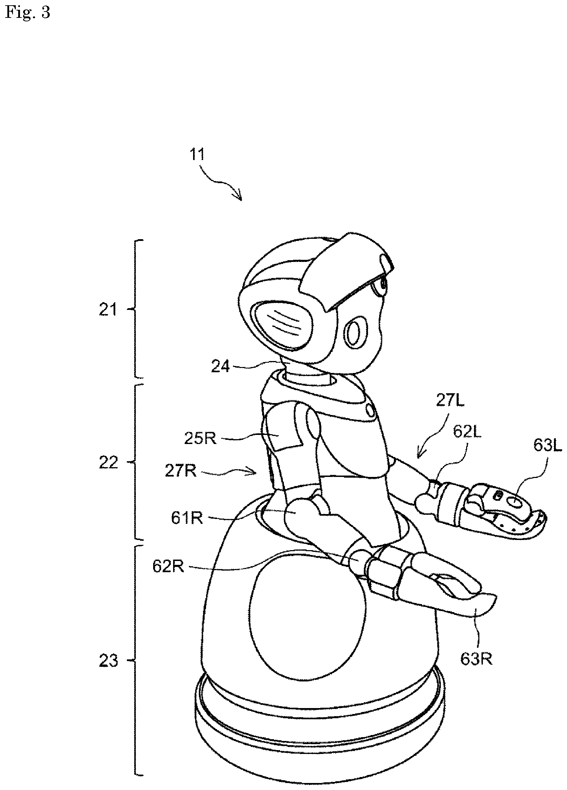

   Fig. 3 is a perspective view of the nursing care robot as viewed from diagonal front right.
[Fig. 4]

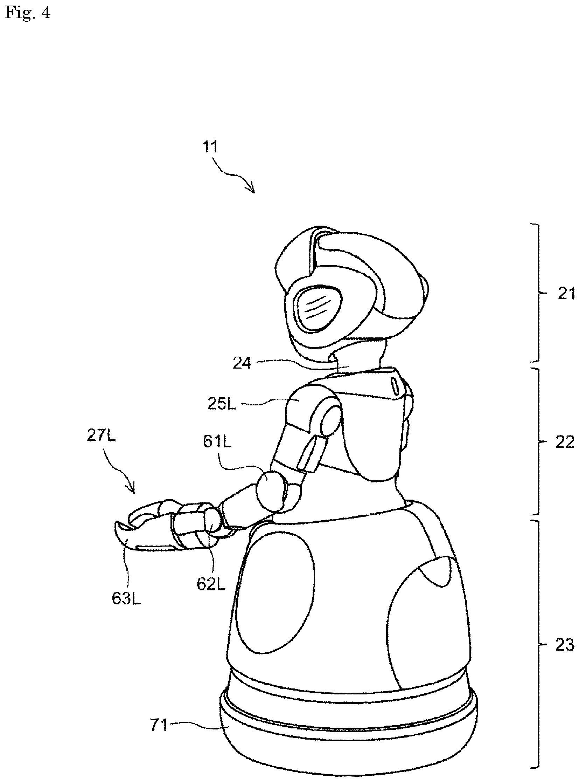

   Fig. 4 is a perspective view of the nursing care robot as viewed from the diagonal rear left.
[Fig. 5]

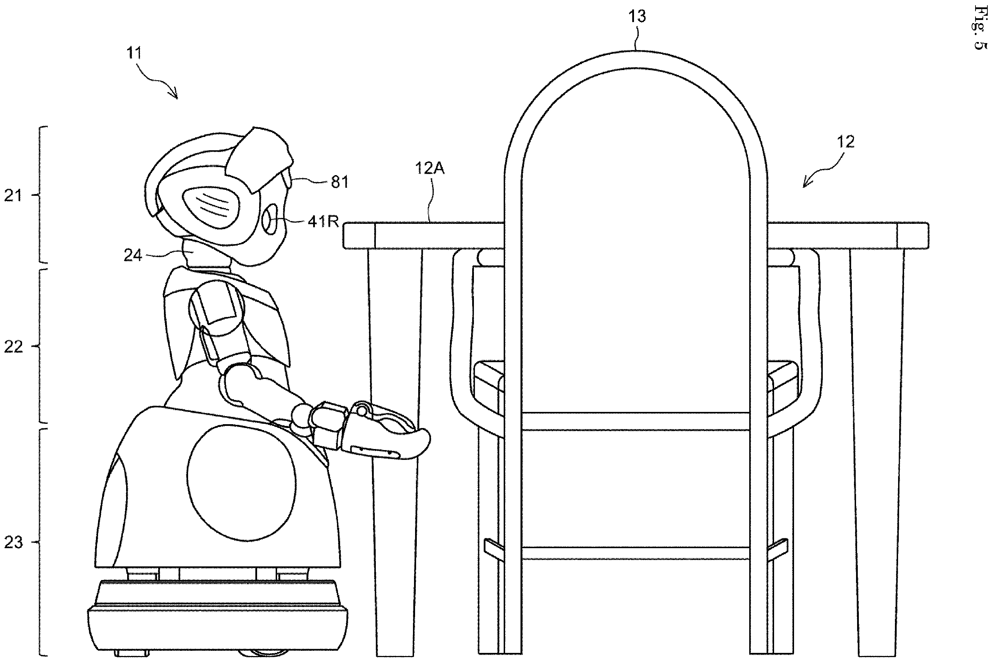

   Fig. 5 is a view of the nursing care robot and a typical table for size comparison.
[Fig. 6]

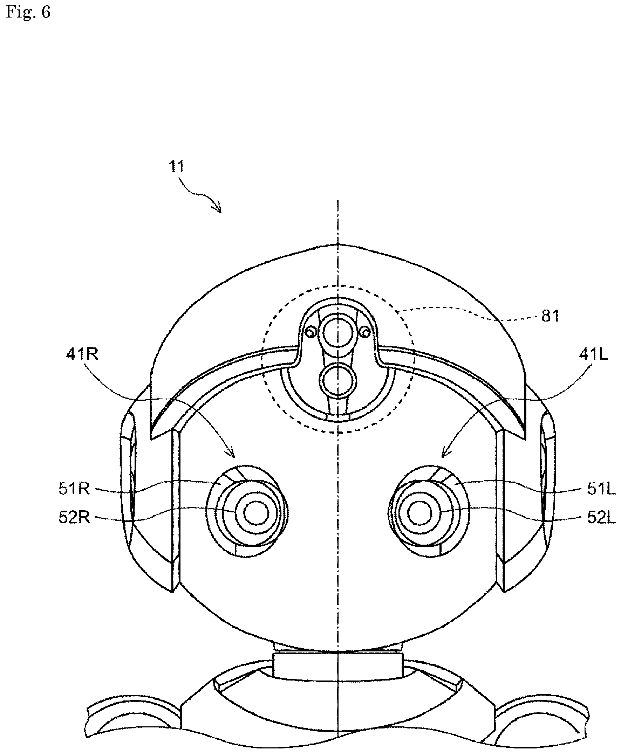

   Fig. 6 is an enlarged view of the head of the nursing care robot.
[Fig. 7]

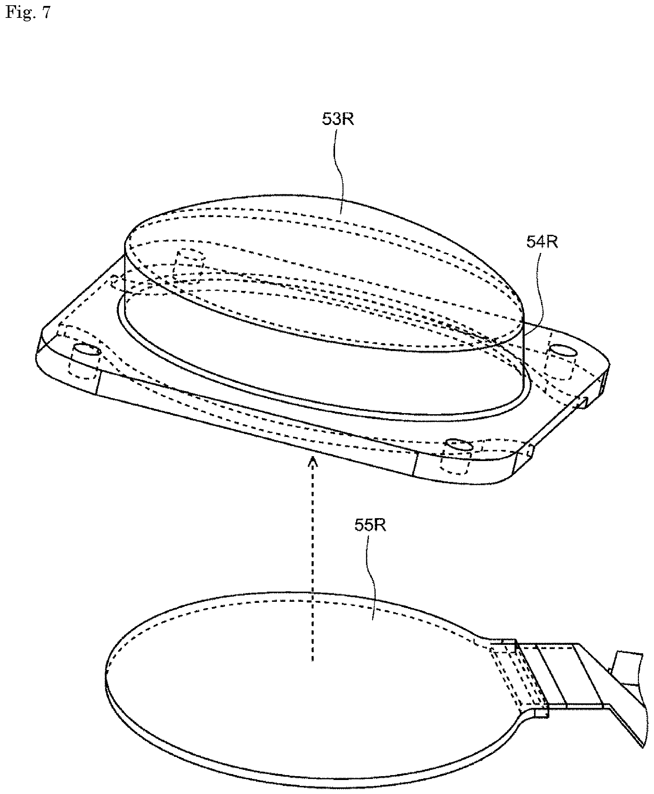

   Fig. 7 is an exploded view of an exemplary internal structure of the eyeball part of the nursing care robot.
[Fig. 8]

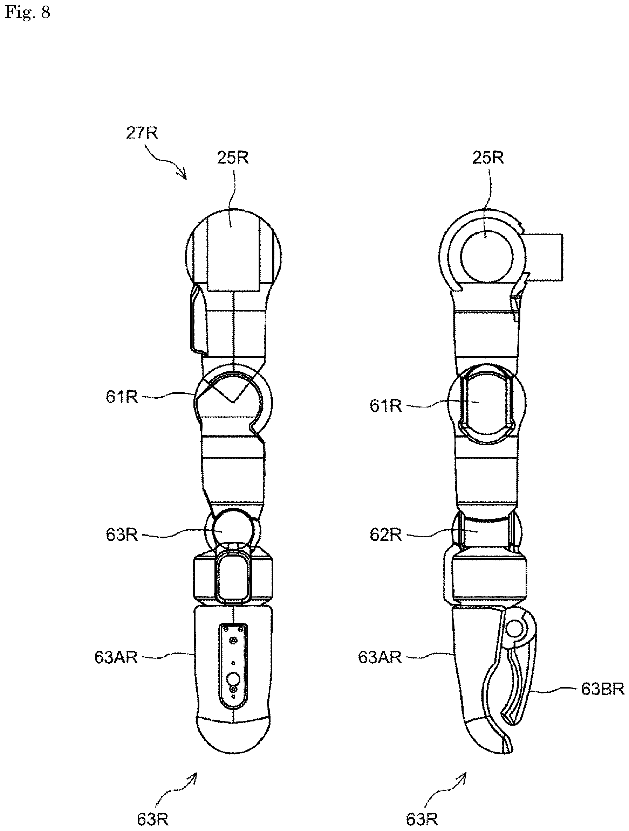

   Fig. 8 is an external view of the arm of the nursing care robot.
[Fig. 9]

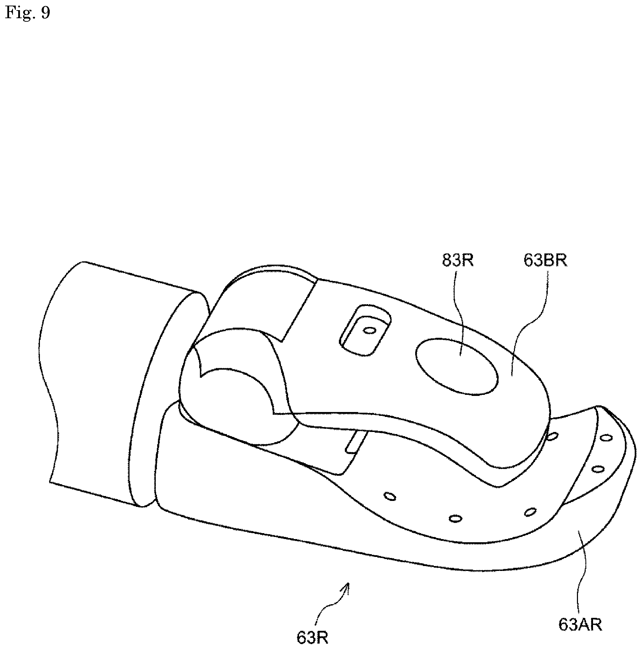

   Fig. 9 is an external view of a hand of the nursing care robot.
[Fig. 10]

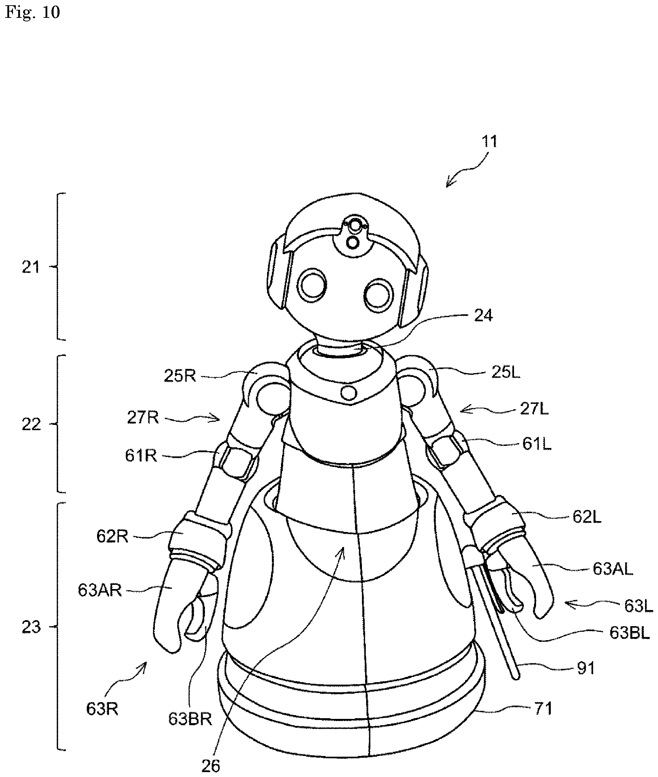

   Fig. 10 is a front view of the nursing care robot mounted with a hand display.
[Fig. 11]

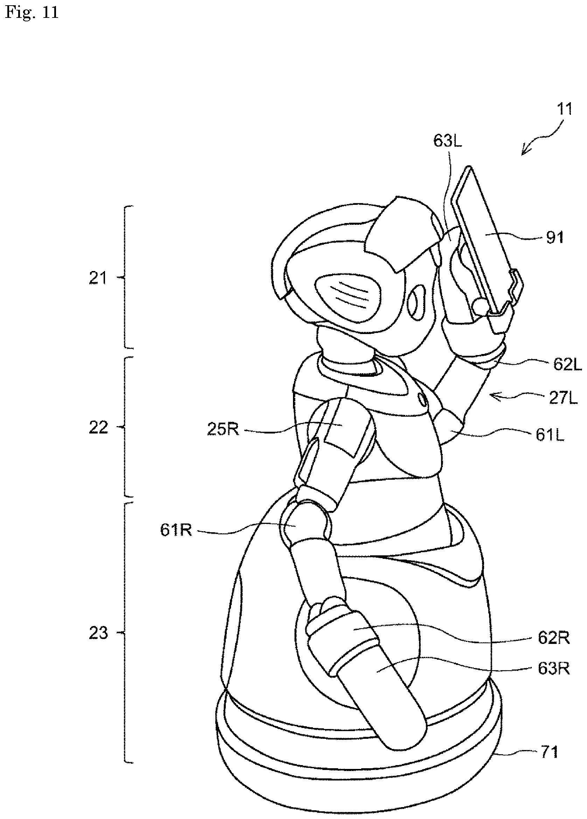

   Fig. 11 is a right side view of the nursing care robot mounted with the hand display.
[Fig. 12]

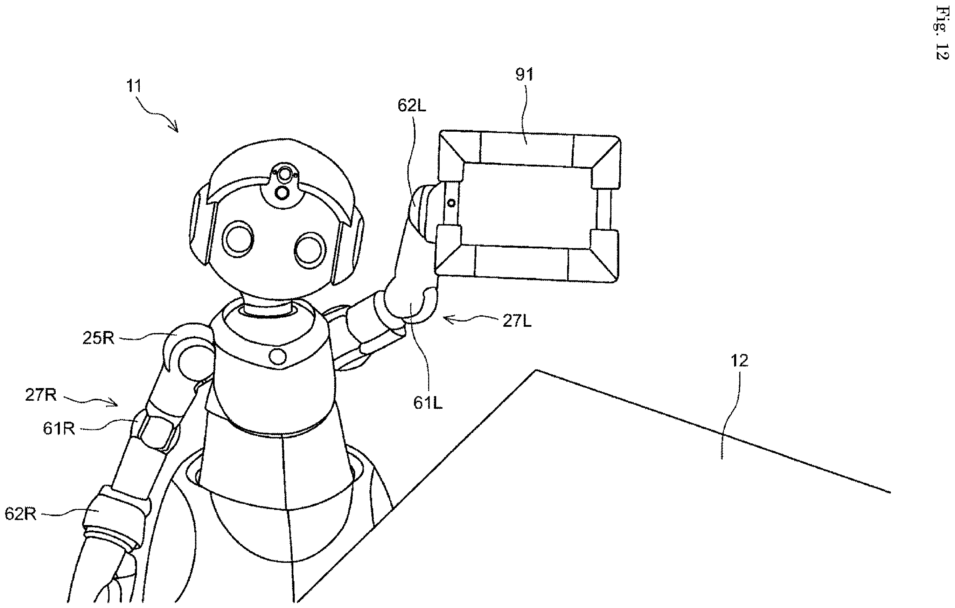

   Fig. 12 is an external view of the upper half body of the nursing care robot mounted with the hand display.
[Fig. 13]

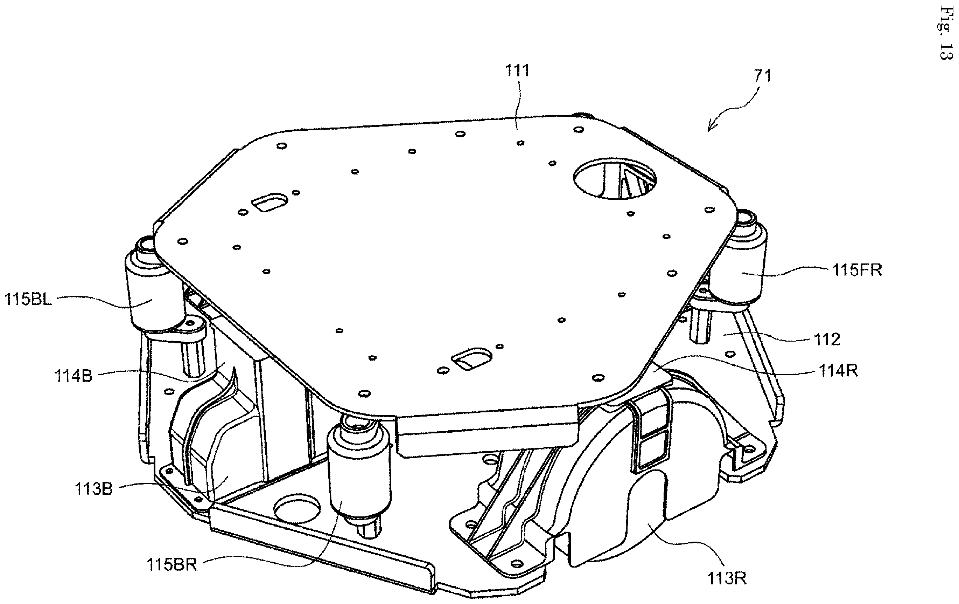

   Fig. 13 is a perspective view of the cart of the nursing care robot as viewed from diagonal rear right.
[Fig. 14]

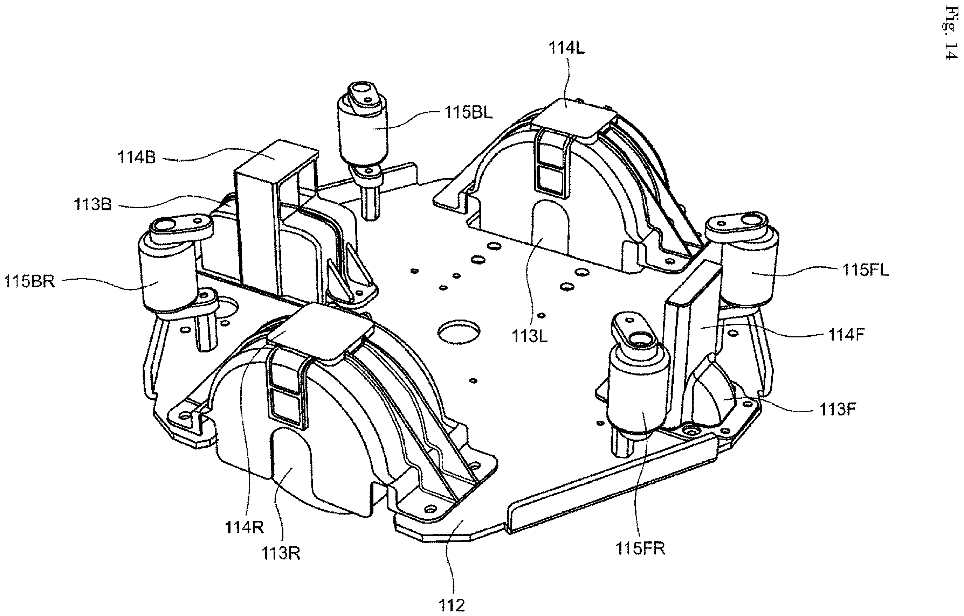

   Fig. 14 is a perspective view of the cart of the nursing care robot removed of the top plate as viewed from the diagonal front right.
[Fig. 15]

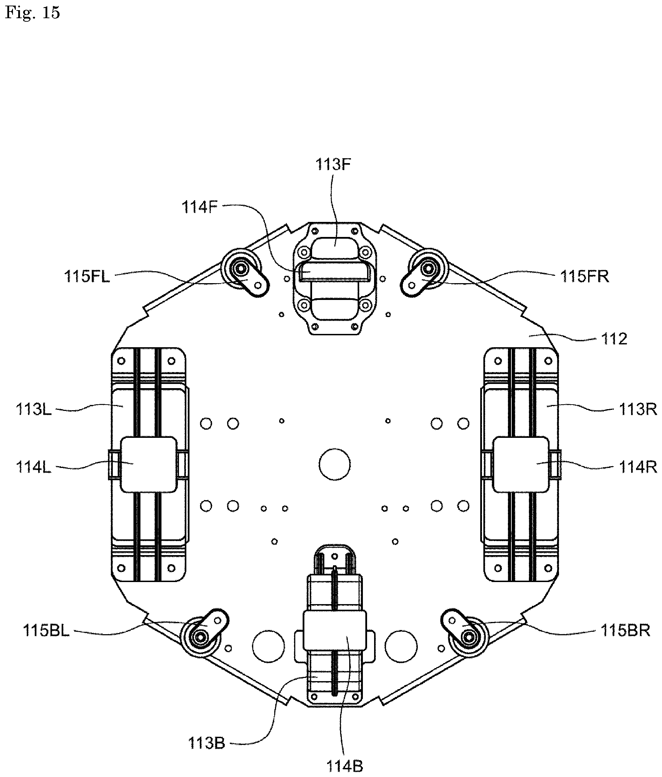

   Fig. 15 is a plan view of the cart removed of the top plate.
[Fig. 16]

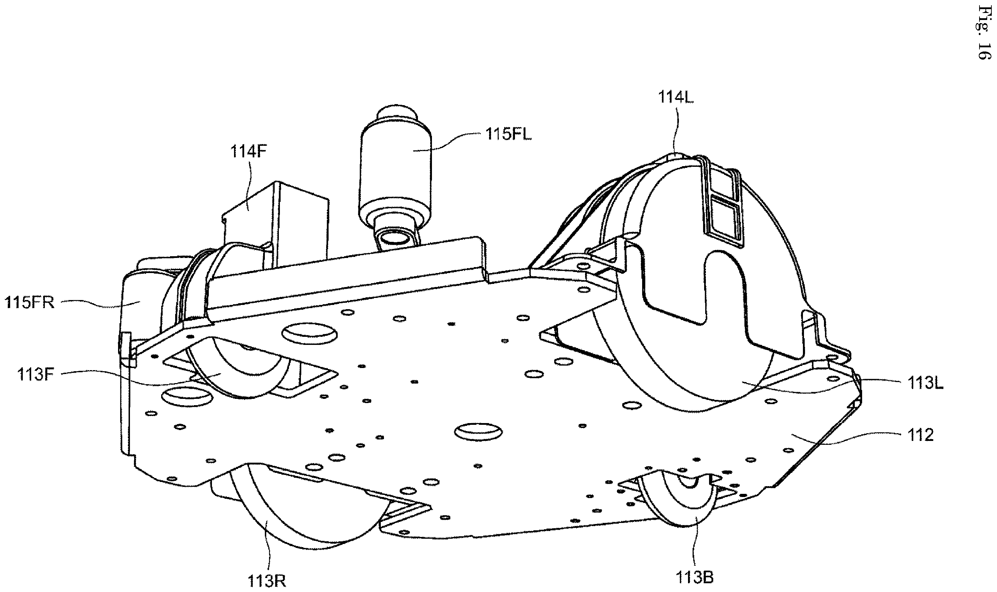

   Fig. 16 is an external view of the cart removed of the top plate as viewed from the diagonal front left and from below.
[Fig. 17]

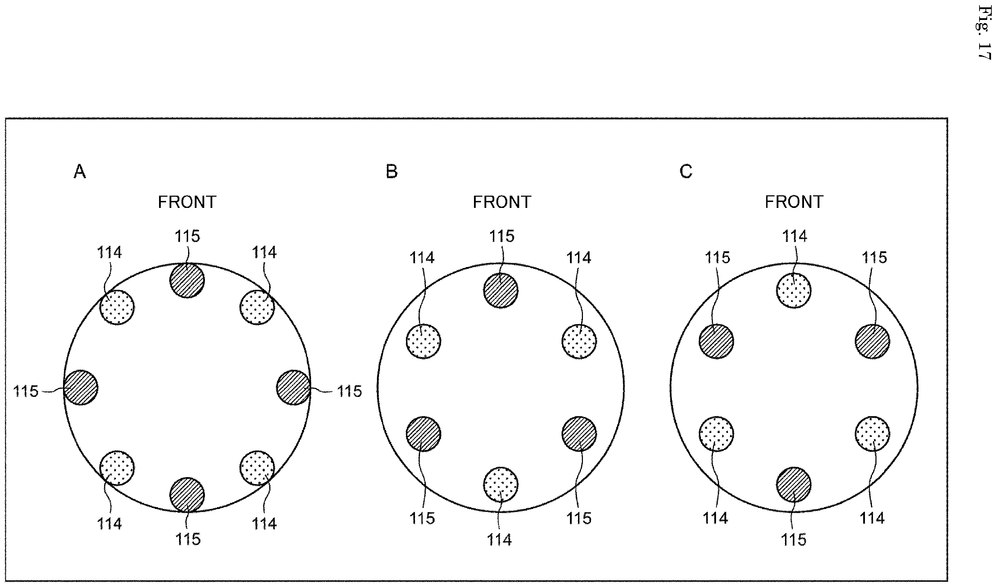

   Fig. 17 is a view of modified examples of the layout of dampers and springs of the cart of the foreign language robot.
[Fig. 18]

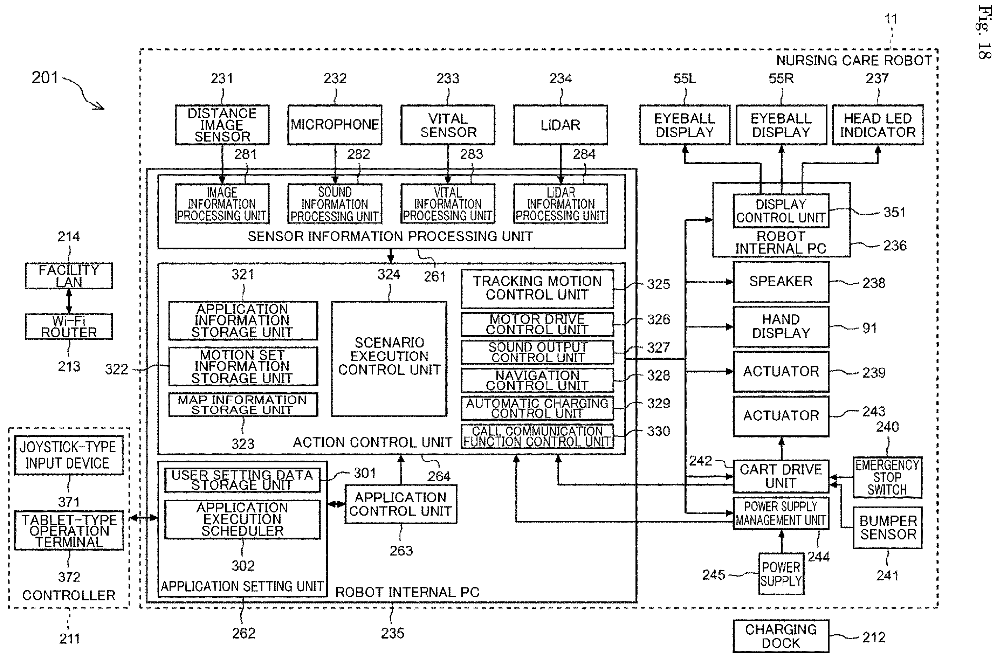

   Fig. 18 is a block diagram of an exemplary configuration of a robot operation system to which the present technology is applied.
[Fig. 19]

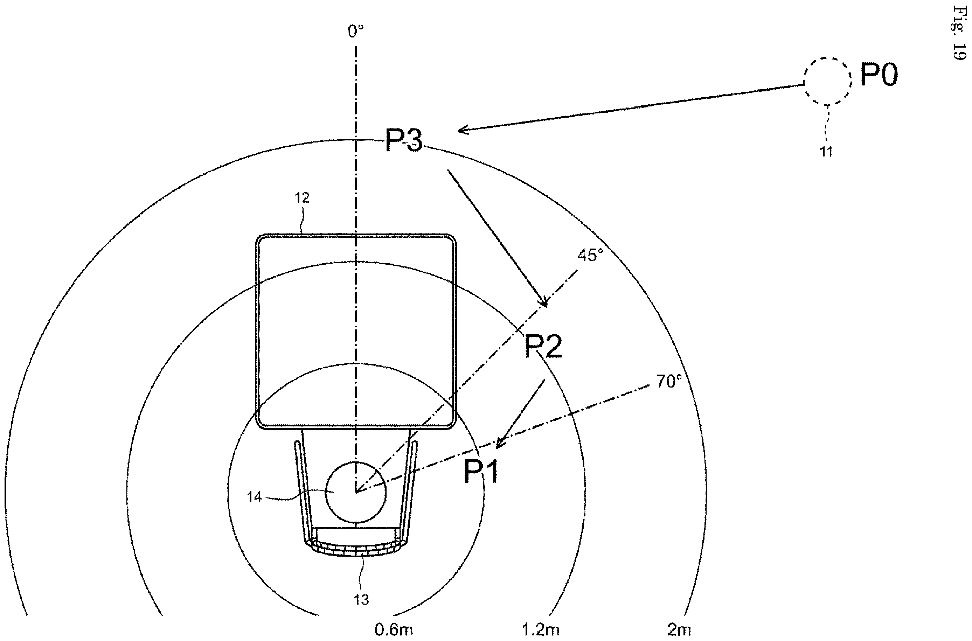

   Fig. 19 illustrates examples of positions between which the nursing care robot moves.
[Fig. 20]

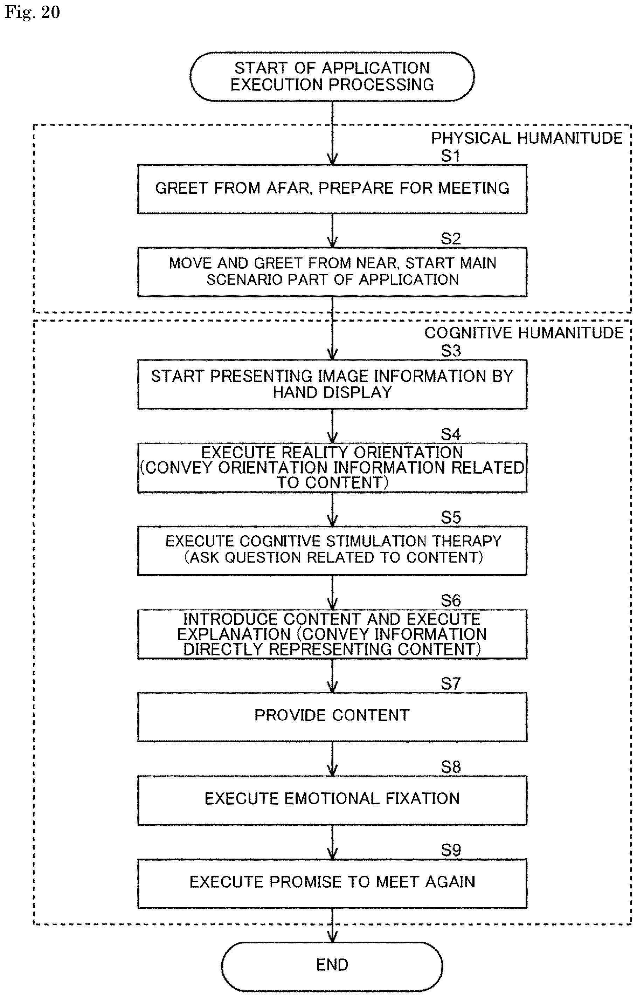

   Fig. 20 is a flowchart for illustrating the basic flow of application execution processing executed by the nursing care robot.
[Fig. 21]

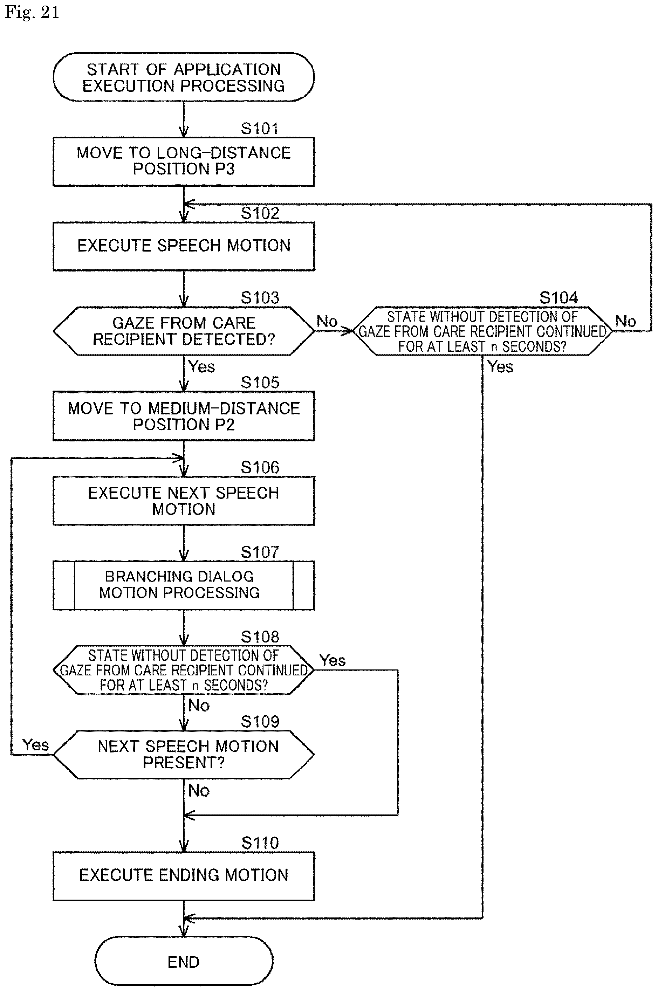

   Fig. 21 is a flowchart for illustrating a first specific example of the application execution processing executed by the nursing care robot.
[Fig. 22]

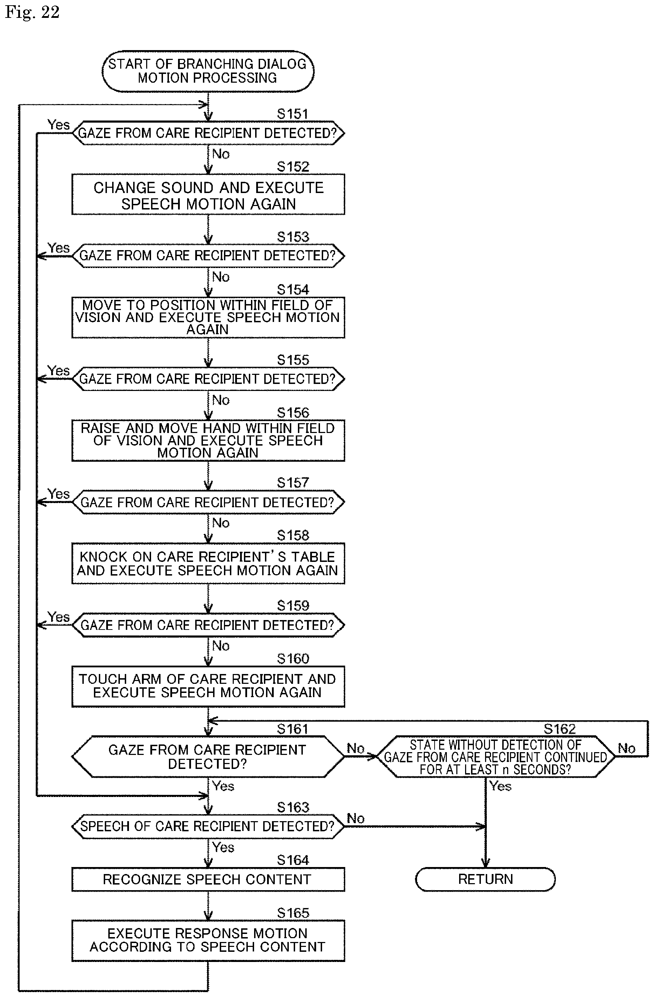

   Fig. 22 is a flowchart for illustrating details of branching dialog processing.
[Fig. 23]

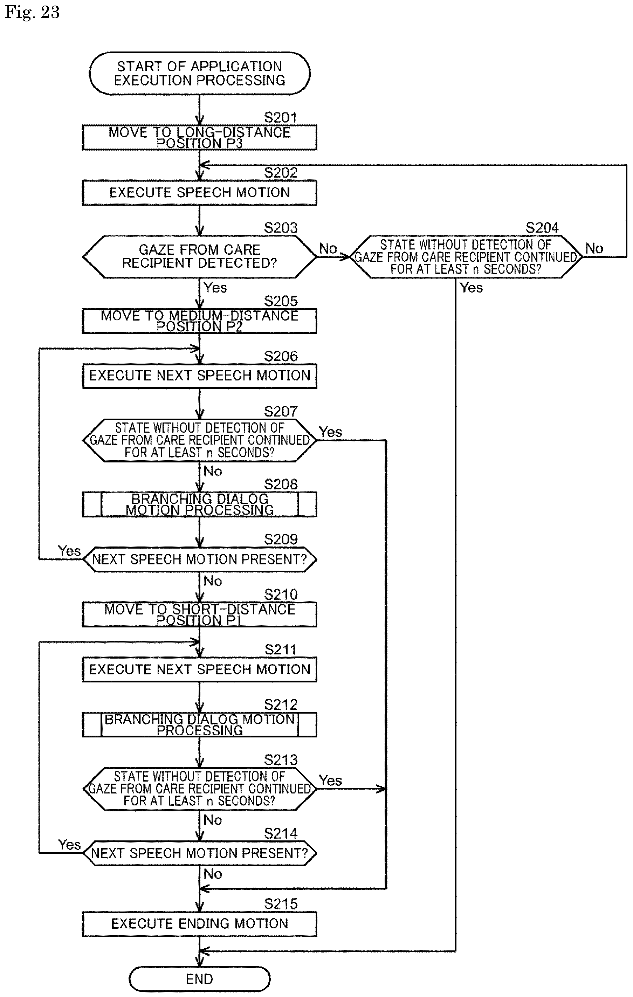

   Fig. 23 is a flowchart for illustrating a second specific example of the application execution processing executed by the nursing care robot.
[Fig. 24]

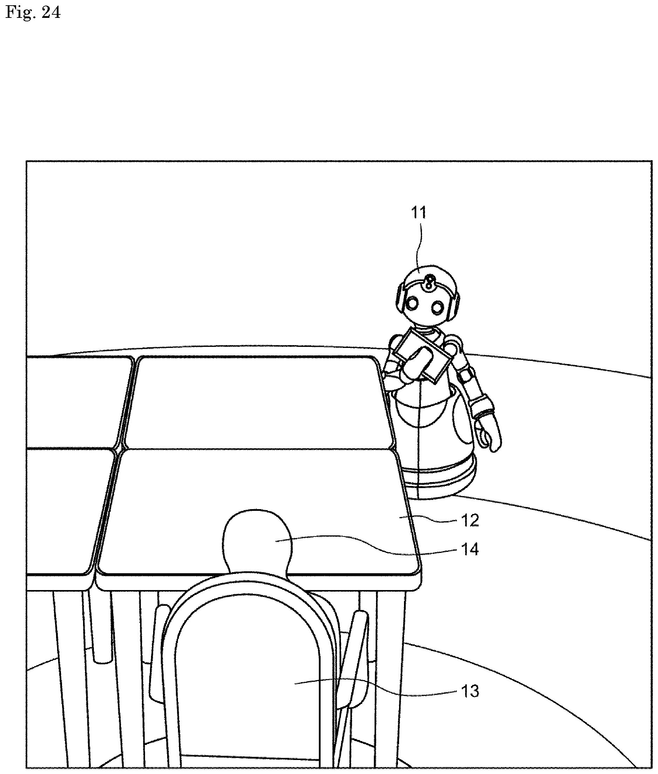

   Fig. 24 is a view for illustrating a specific example of a vital measurement application.
[Fig. 25]

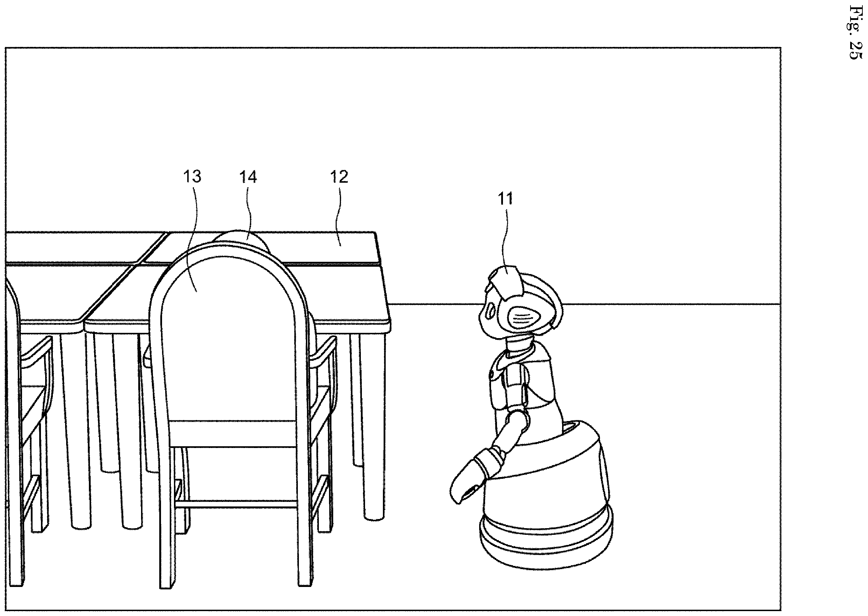

   Fig. 25 is a view for illustrating the specific example of the vital measurement application.
[Fig. 26]

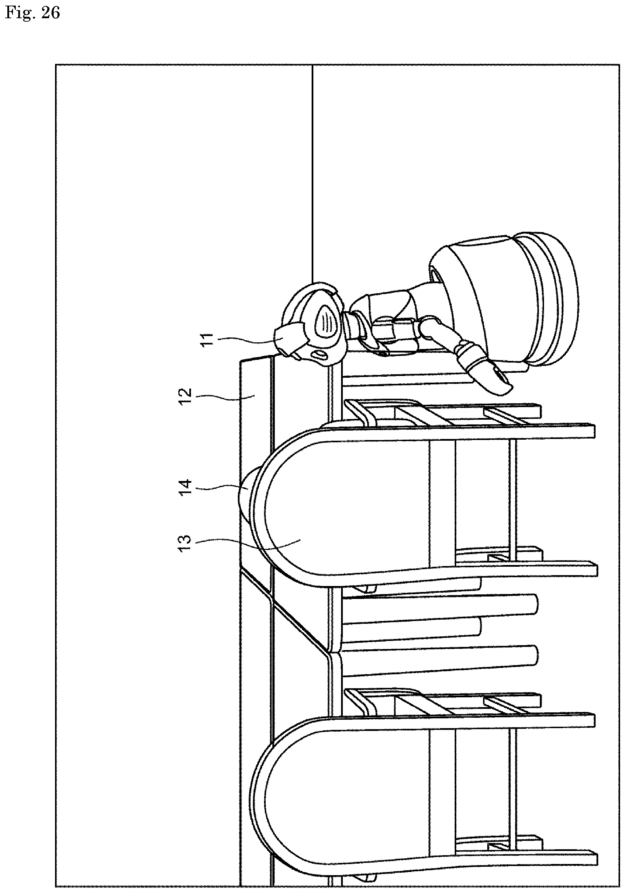

   Fig. 26 is a view for illustrating the specific example of the vital measurement application.
[Fig. 27]

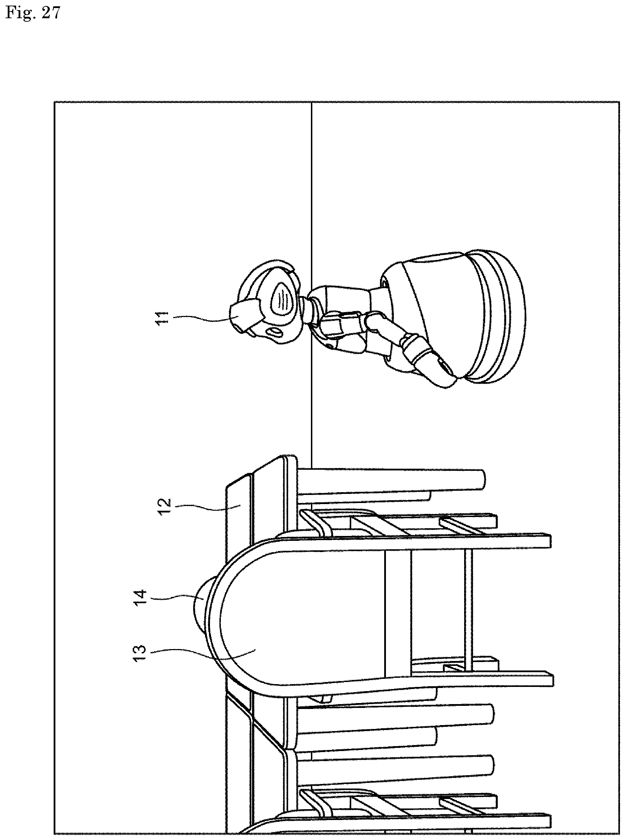

   Fig. 27 is a view for illustrating the specific example of the vital measurement application.
[Fig. 28]

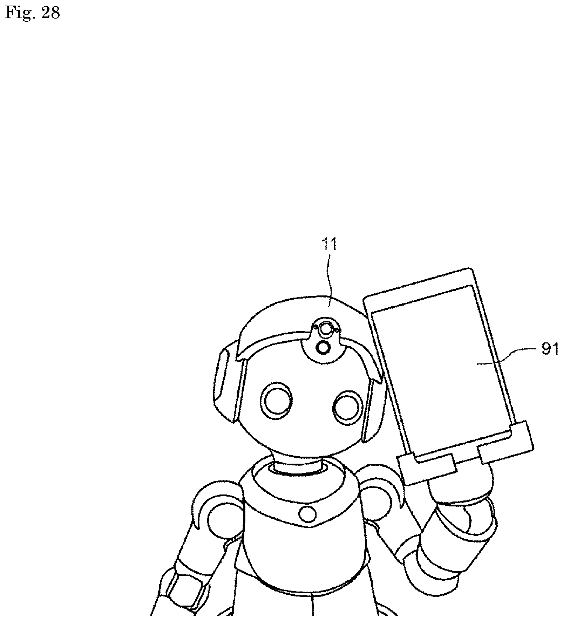

   Fig. 28 is a view for illustrating a specific example of a music therapy application.
[Fig. 29]

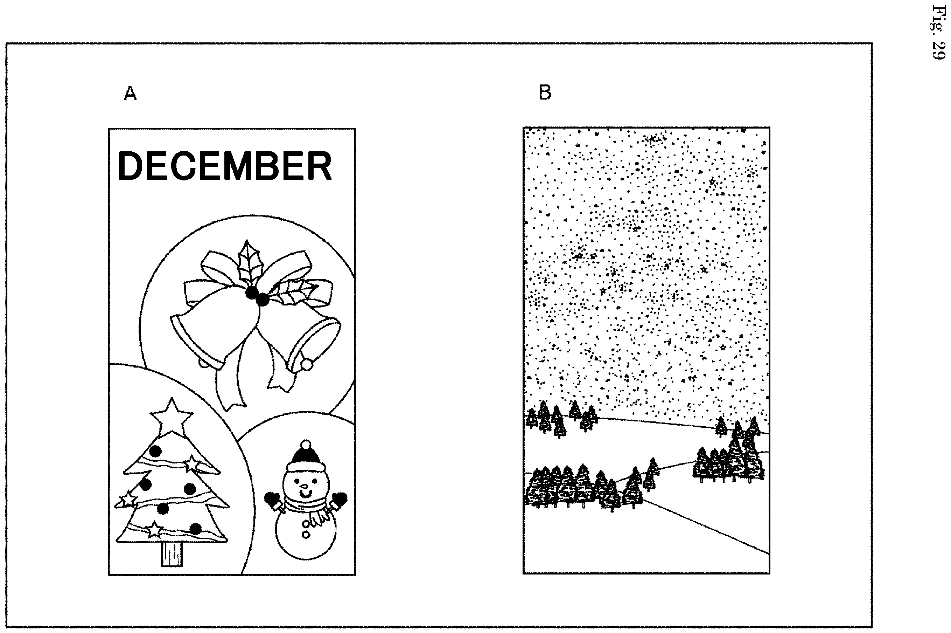

   Fig. 29 is a view for illustrating the specific example of the music therapy application.
[Fig. 30]

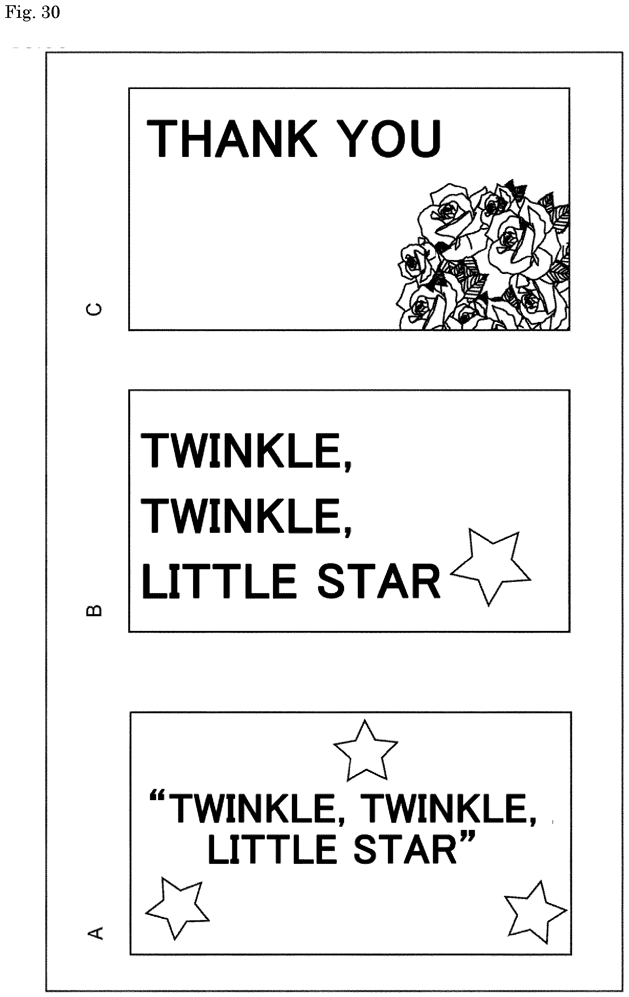

   Fig. 30 is a view for illustrating a specific example of the music therapy application.
[Fig. 31]

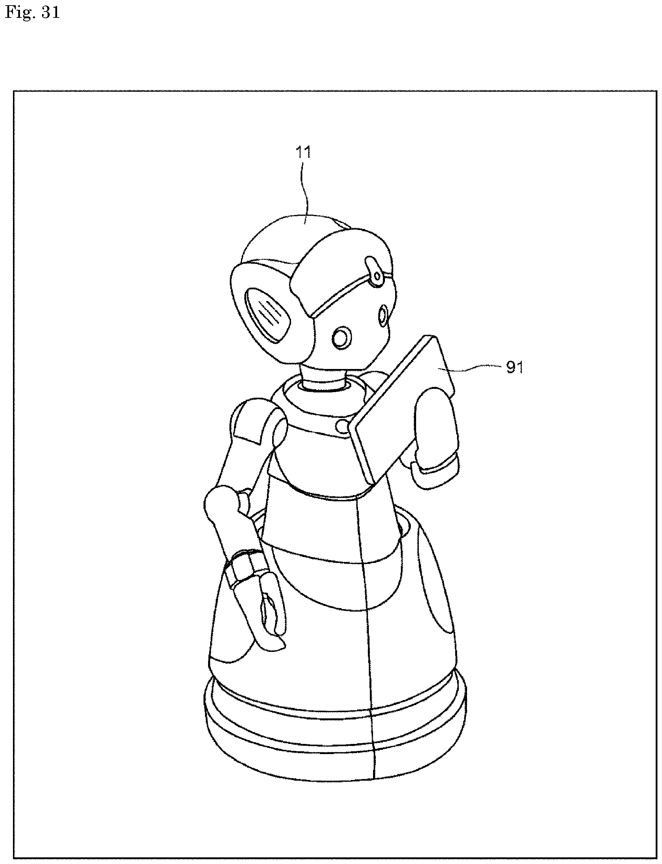

   Fig. 31 is a view for illustrating a specific example of a telephone application.
[Fig. 32]

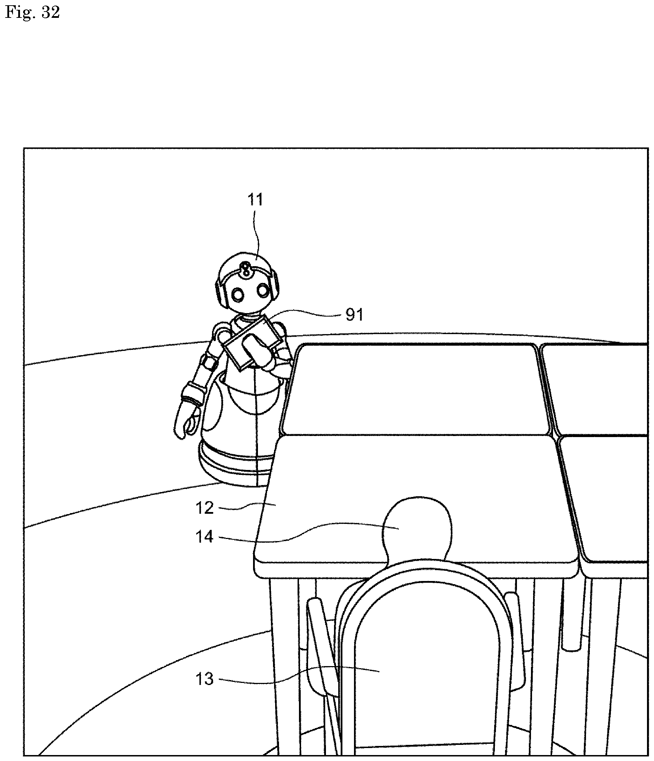

   Fig. 32 is a view for illustrating the specific example of the telephone application.
[Fig. 33]

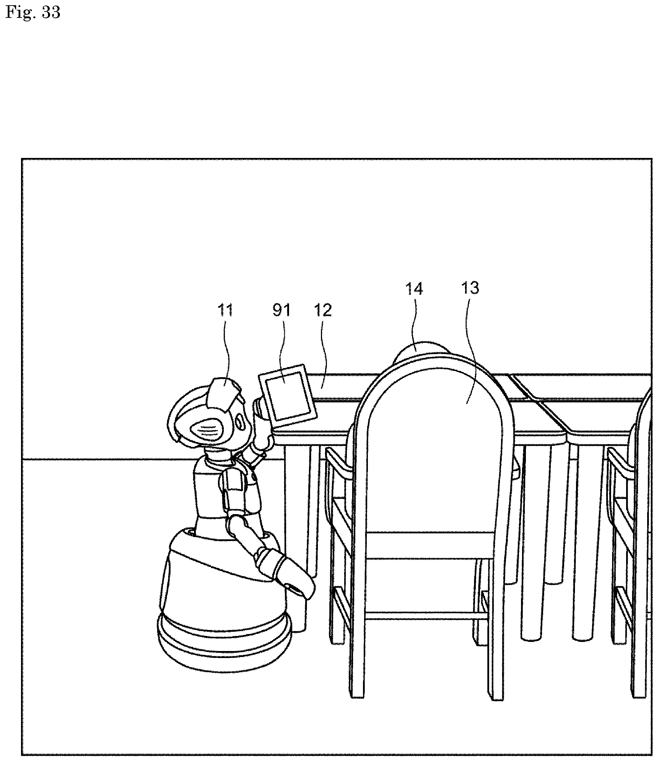

   Fig. 33 is a view for illustrating the specific example of the telephone application.
[Fig. 34]

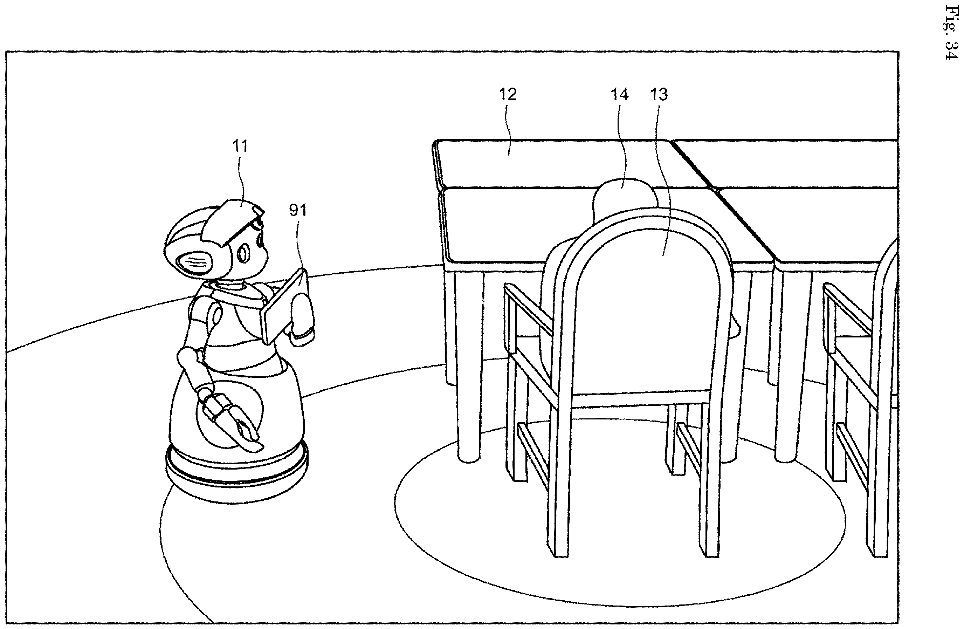

   Fig. 34 is a view for illustrating the specific example of the telephone application.
[Fig. 35]

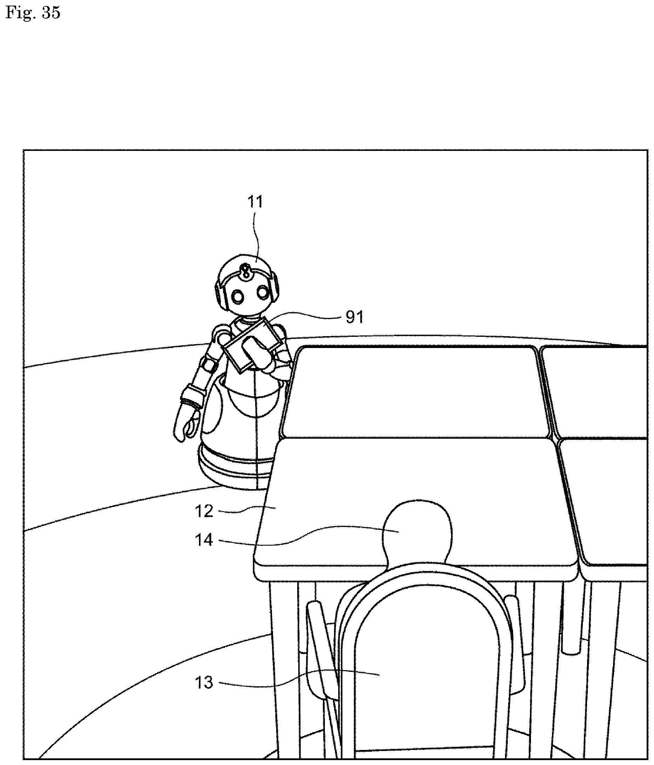

   Fig. 35 is a view for illustrating the specific example of the telephone application.
[Fig. 36]

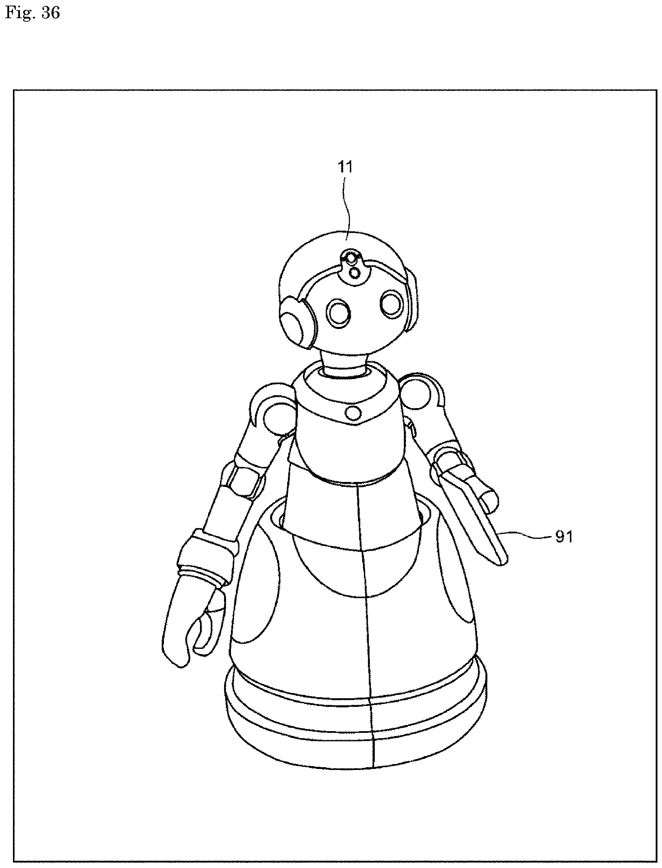

   Fig. 36 is a view for the illustrating the specific example of the telephone application.
[Fig. 37]

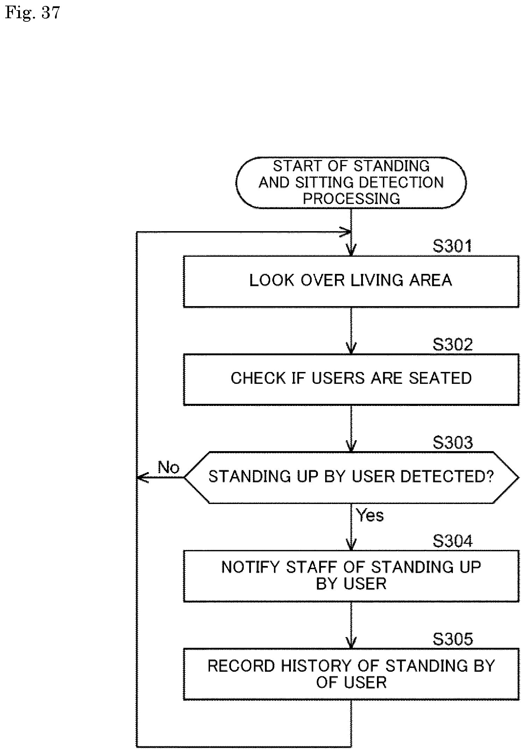

   Fig. 37 is a flowchart for illustrating standing and sitting detection processing executed by the nursing care robot.
[Fig. 38]

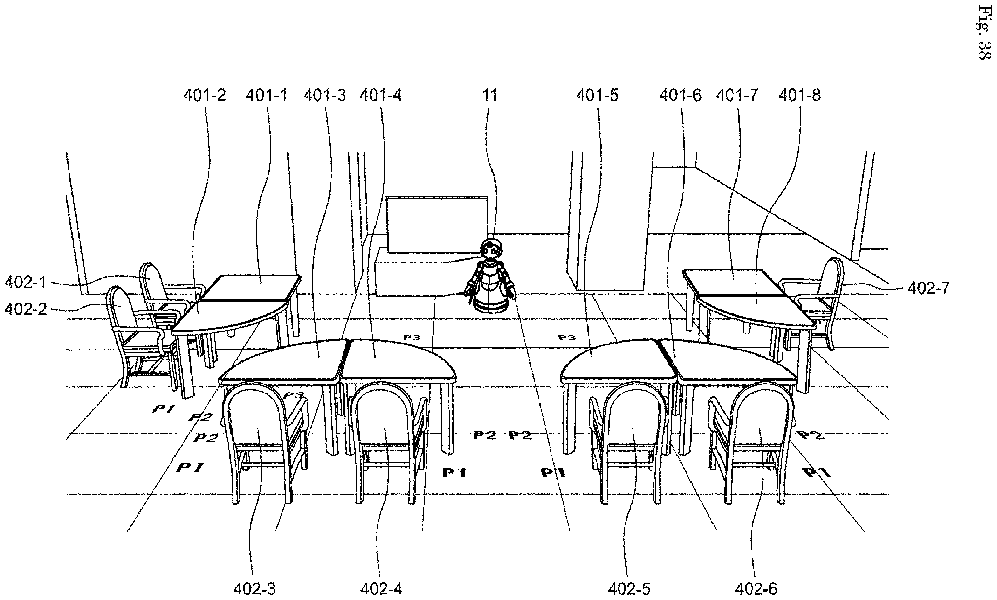

   Fig. 38 is a view of an example of arrangement of tables and chairs in a living area.
[Fig. 39]

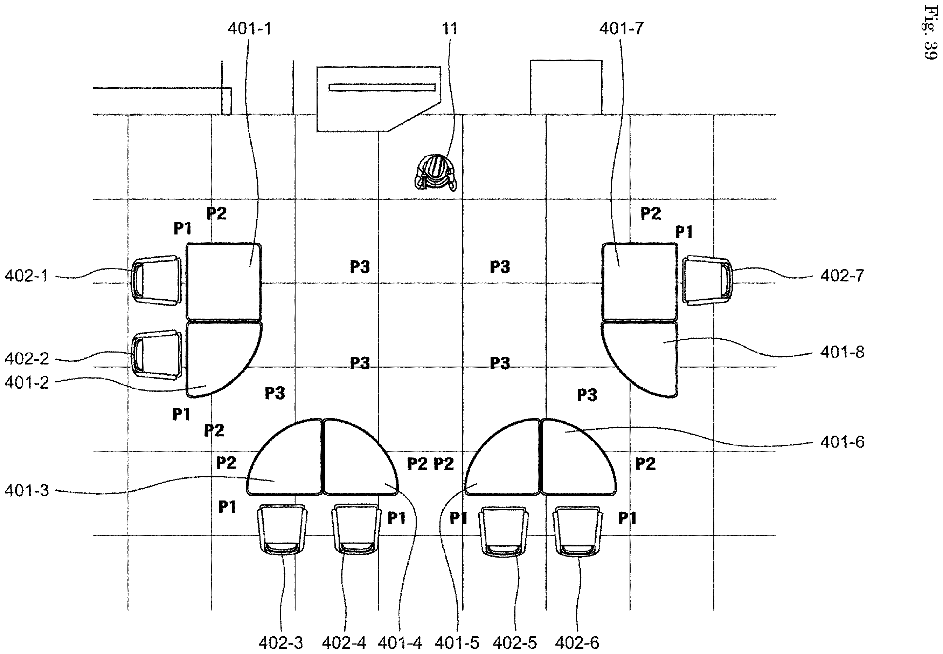

   Fig. 39 is a view of the example of arrangement of tables and chairs in the living area.
[Fig. 40]

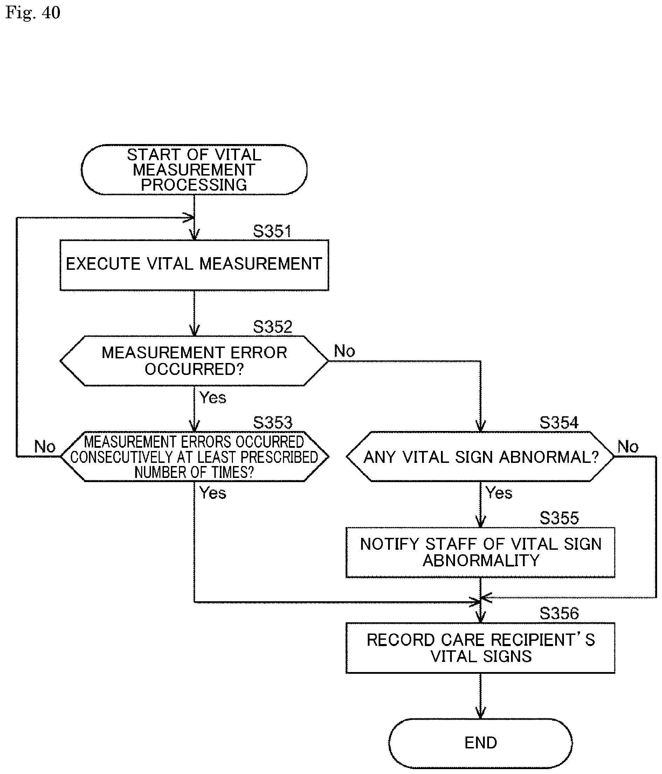

   Fig. 40 is a flowchart for illustrating vital measurement processing executed by the nursing care robot.
[Fig. 41]

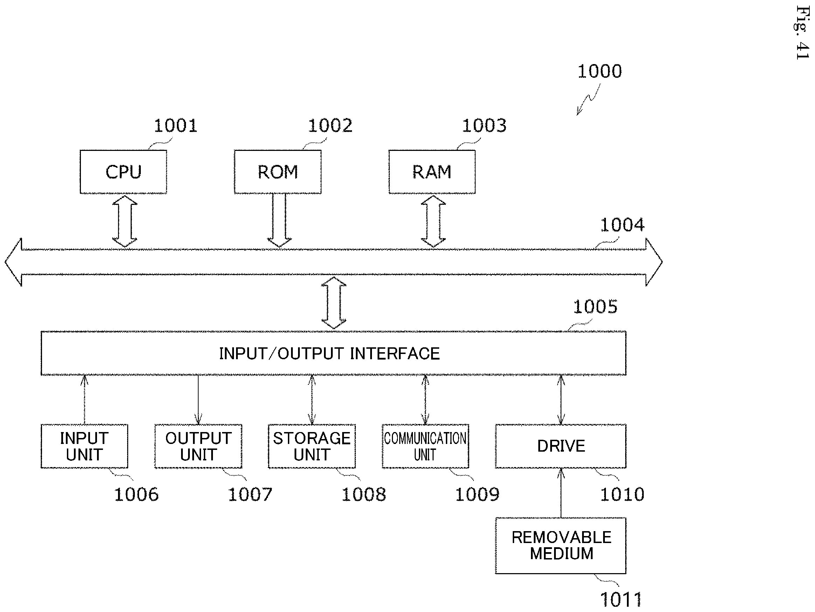

   Fig. 41 is a block diagram of an exemplary configuration of a computer.

### [Description of Embodiments]

Hereinafter, embodiments of the present technology will be described in detail with reference to the drawings. Here, the description will be given in the following order.
0. Background of present technology
1. Embodiment
2. Modification
3. Others

### <<0. Background of present technology>>

In care facilities such as nursing homes, for example, primarily due to staff shortages, the absence of staff from the living areas and the waiting periods for people under care, such as those with dementia (hereinafter simply referred to as "care recipients"), can make the care recipients prone to mental instability. For example, the care recipients tend to become restless, feel lonely, or get angry.

In addition, care workers having a high workload have difficulty in providing sufficient time for individual care such as music therapy and conversation, which aim to prevent the progression of dementia, prevent destabilization, and improve the quality of life (QOL) of care recipients. Furthermore, in nursing care facilities, activities aimed to enhance the quality of life (QOL) of the care recipients are lacking due to staff shortages and restrictions on visits due to the COVID-19 pandemic.

Meanwhile, people with dementia may not readily accept music therapy simply by playing music using for example karaoke, music players, TV sets, or tablet devices, as part of the music therapy. On the other hand, it might be possible to explain about songs using images, but it is difficult for people with dementia to perceive the existence in the images as a subject that talks to them.

People with dementia may have difficulty in managing and operating video phones on their own.

Meanwhile, the present technology is directed to improving the receptiveness of care recipients to various types of care such as music therapy conducted by a nursing care robot.

Specifically, for example, as will be described, a nursing care robot executes an application for providing care to a care recipient using Humanitude. For example, a nursing care robot executes physical Humanitude and cognitive Humanitude before providing care to a care recipient.

Physical Humanitude is a physical approach that stepwise reduces the physical distance between the nursing care robot and the care recipient.

Cognitive Humanitude is a cognitive approach that stepwise reduces the cognitive distance of a care recipient to content to be provided in an application for providing care to the care recipient.

The content to be provided to care recipients here includes those for specific care performed to the care recipient and items used for providing care. Examples of items used for providing care include moving images, music, and other kinds of content used in music therapy as well as devices used in for providing care. In some cases, for example, content may be provided to care recipients as the nursing care robot itself executes the content, while in other cases, content may be provided to care recipients as the nursing care robot presents the content.

### <<1. Embodiment>>

Referring to Figs. 1 to 36, an embodiment of the present technology will be described.

### <Exemplary configuration of nursing care robot 11>

Referring to Figs. 1 to 17, a nursing care robot 11 according to an embodiment of the present technology will be described.

The nursing care robot 11 is a humanoid mobile manipulator robot capable of executing various applications for various types of care, condition observation, communication, and peripheral work tasks, with a quality that ensures high receptiveness among care recipients.

For example, the nursing care robot 11 executes various applications for performing nursing care actions according to a scheduler and robot operation settings for each care recipient created under the discretion of nursing care staff. Examples of applications that the nursing care robot 11 can execute include those for greeting and calming the care recipient, vital measurement, music therapy, and telephone calls. The nursing care robot 11 is configured to be able to execute applications aimed to prevent destabilization of care recipients and helping the care recipients to regulate their daily rhythm.

### <Exemplary configuration of external appearance of nursing care robot 11>

Figs. 1 to 9 illustrate an exemplary configuration of external appearance of the nursing care robot 11. Fig. 1 at A is a front view of the nursing care robot 11. Fig. 1 at B is a left side view of the nursing care robot 11. Fig. 2 is a rear view of the nursing care robot 11. Fig. 3 is a perspective view of the nursing care robot 11 as viewed from the diagonal front right. Fig. 4 is a perspective view of the nursing care robot 11 as viewed from the diagonal rear left. Fig. 5 is a view of the nursing care robot 11 and a typical table 12 for size comparison. Fig. 6 is an enlarged view of the head 21 of the nursing care robot 11. Fig. 7 is an exploded view of an exemplary internal configuration of the eyeball part 41R of the nursing care robot 11. Fig. 8 is an external view of the arm 27R of the nursing care robot 11. Fig. 9 is an external view of the hand 63R of the nursing care robot 11.

The nursing care robot 11 for example has an appearance of a child as a model according to the concept of a grandchild robot.

The nursing care robot 11 has a head 21, a chest 22, and a base part 23 that supports the chest 22. The base part 23 includes, for example, a cart 71 capable of moving in all directions at the bottom. This allows the nursing care robot 11 to move in all directions.

The nursing care robot 11 has an arm 27L attached to the left side and upper part of the chest 22, and an arm 27R attached to the right side and upper part of the chest 22. The nursing care robot 11 has a movable neck 24 provided between the head 21 and the chest 22, and having a neck joint axis 24C. The nursing care robot 11 has a movable shoulder part 25L located between the chest 22 and the arm 27L and having a shoulder joint axis 25LC and a movable shoulder part 25R located between the chest 22 and the arm 27R and having a shoulder joint axis 25RC. The nursing care robot 11 also has a movable waist 26 provided at the lower part of the chest 22 and having a waist joint axis 26C.

The height of the nursing care robot 11 is such that a seated care recipient can effortlessly look down on the robot from a seated position. The nursing care robot 11 has such a height that the robot can overlook the top of a typical table 12, as shown in Fig. 5.

As shown in Fig. 6, the head 21 has eyeball parts 41L and 41R. The eyeball part 41L has a white eye part 51L and a black eye part 52L arranged within the white eye part 51L. The eyeball part 41R has a white eye part 51R and a black eye part 52R arranged within the white eye part 51R, similarly to the eyeball part 41L.

As shown in Fig. 7, the eyeball part 41R has a transparent solid cylindrical part 54R having first and second end surfaces. The eyeball part 41R has a flat eyeball display 55R provided on the first end surface side (lower side in Fig. 7) of the cylindrical part 54R to display the movement of the black eye part 52R. The eyeball part 41 has a hemispherical transparent spherical part 53R provided on the second end surface side (upper side in Fig. 7) of the cylindrical part 54R to emit display light entered from the eyeball display 55R through the cylindrical part 54R. The spherical part 53R forms a hemispherical transparent spherical lens. The outer circumferential shape of the spherical part 53R is configured to form the white eye part 51R.

The outer circumferential surface of the cylindrical part 54R is non-transparent to prevent light from entering, so that the display image on the eyeball display 55R visible from the spherical part 53R is clear and undistorted. The spherical part 53R is arranged with a gap from the eyeball display 55R, which creates a sense of depth and a three-dimensional effect. The center of the sphere on the spherical part 53R is designed as the virtual rotation center of the eyeball part, and the movement of the black eye part 52R displayed on the eyeball display 55R is controlled with reference to the center of the sphere of the spherical part 53R.

As described above, the eyeball part 42R is displayed on a built-in sphere that, unlike a flat display, can be seen from any angle and appears to be moving, without distortion, making it possible to recreate an actual eyeball-like appearance. The center of the black eye part 52R matches the center of the spherical part 53R, so that there is no sense of awkwardness in the thickness or shape of the sphere. Furthermore, reflections of ambient light on the surface of the spherical part 53R allow for natural and real-time representation of highlights in the pupil.

Although not shown, the eyeball part 41L also has a spherical part 53L, a cylindrical part 54L, and an eyeball display 55L, and is configured to be symmetrical with the eyeball part 41R.

The nursing care robot 11 performs human recognition and face recognition using a head sensor 81, and gazes at a care recipient by controlling the position of the black eye part 52L of the eyeball part 41L, the position of the black eye part 52R of the eyeball part 41R, and the axes (roll, pitch, yaw) of the neck 24. Specifically, the black eye parts 52L and 52R track down the position of the care recipient in the up-down and left-right directions to gaze the care recipient. The distance to the care recipient is expressed using the convergence angle (crossed eyes, close eyes) between the black eye parts 52L and 52R. This makes it easier for the care recipient to recognize where the nursing care robot 11's gaze is directed (especially in terms of depth perception).

Hereafter, when there is no need to distinguish between the left and right eyeball parts 41L and 41R individually, these will simply be referred to as eye parts 41. When there is no need to distinguish between the white eye parts 51L and 51R individually, these will simply be referred to as white eye parts 51. When there is no need to distinguish between the black eye parts 52L and 52R individually, these will simply be referred to as black eye parts 52. When there is no need to distinguish between the spherical parts 53L and 53R, they will simply be referred to as the spherical parts 53. When there is no need to distinguish between the cylindrical parts 54L and 54R, they will simply be referred to as the cylindrical parts 54. When there is no need to distinguish between the eyeball displays 55L and 55R, they will simply be referred to as the eyeball displays 55.

As shown in Fig. 8, the arm 27R has an elbow 61R, a wrist 62R, and a hand 63R. The elbow 61R has a pitch axis. The wrist 62R has a yaw axis.

The hand 63R has a part 63AR corresponding to parts other than the thumb and a part 63BR corresponding to the thumb. The part 63BR is opposite to the part 63AR, and by moving the part 63BR, it is possible to grip an object with the hand 63R.

Although not shown, the arm 27L is also configured similarly to the arm 27R, and has an elbow 61L, a wrist 62L, and a hand 63L. The hand 63L has parts 63AL and 63BL, similarly to the hand 63R.

Hereinafter, when there is no need to distinguish between the arm 27L and arm 27R individually, they will be simply referred to as arms 27. When there is no need to distinguish between the elbows 61L and 61R individually, they will be simply referred to as elbows 61. When there is no need to distinguish between the wrists 62L and 62R individually, they will be simply referred to as wrists 62. When there is no need to distinguish between the hands 63L and 63R individually, they will be simply referred to as hands 63.

The head sensor 81 is provided at the front upper part of the head 21. The head sensor 81 has, for example, a distance image sensor, a microphone, and a LiDAR (Light Detection and Ranging) device.

The head sensor 81 is configured so that the sensing direction is approximately the same as the direction of the nursing care robot 11's line of sight, so that for example, Humanitude motions, face tracking motions can be performed.

For example, the nursing care robot 11 can perform human recognition and face recognition using the head sensor 81 and can carry out interactions that track the care recipient's eyes.

For example, the height of a typical table 12 is about 700 mm, while the height of a typical table 12 in a nursing care facility is about 660 mm. For example, as shown in Fig. 5, the head sensor 81 is positioned so that the sensor can look over the top of the tabletop 12A of the table 12. The head 21 is positioned so that the head can look up at the face of the care recipient while the care recipient sits in a chair 13 (sitting posture). The head sensor 81 is installed at a high position of the head 21 (for example, at a height of around 760 mm) facing upwards at an angle of around 5°. The head sensor 81 is positioned so that the head sensor does not protrude too far from the outer diameter line of the head 21.

In this way, the nursing care robot 11 can recognize objects on the standard table 12 or the face of the care recipient in a sitting position over the table 12 as shown in Fig. 5. The nursing care robot 11 is capable of recognizing the face of a care recipient in a sitting position on a standard bed, and of recognizing the face of a care recipient in a supine position on a standard bed. The nursing care robot 11 is capable of recognizing the face of a care recipient in a sitting position at close range, looking up at the care recipient, and of recognizing the face of a care recipient in an upright position at close range, looking up at the care recipient.

A chest sensor 82 is provided in the upper front of the chest 22. The chest sensor 82 includes, for example, a non-contact vital sensor. Examples of the non-contact vital sensors include a temperature sensor, a heart rate sensor, and a respiration sensor.

The chest sensor 82 is installed at an upward angle of about 10° at the front upper position of the chest 22 (for example, at a height of about 537 mm). This allows the chest sensor 82 to measure without being affected by the motion of the head 21. The chest sensor 82 can reduce the blind spots created by the arm 27 during manipulation. The chest sensor 82 enables vital sensing for example from the face of a care recipient in a sitting position, the face of a care recipient in an upright position at a distance (e.g., about 2 m), and the face of a care recipient in a supine position at a close distance. The chest sensor 82 enables constant sensing of changes in the state of a care recipient during application execution.

The hand 63R is provided with a hand sensor 83R. The hand sensor 83R includes for example a contact-type vital sensor. Examples of the contact-type vital sensors include a heart rate sensor, a blood pressure sensor, and an oxygen saturation sensor.

The hand sensor 83R is, for example, as shown in Fig. 9, provided on the outside of the part 63BR of the hand 63R. This prevents the hand 63R from squeezing the care recipient's hand during vital sensing.

In addition, the hand sensor 83R does not allow the nursing care robot 11 to go and place the hand sensor 83R on the care recipient by itself, but rather allows the care recipient to place their hand on it or hold it, thereby enabling vital sensing. This is an interface that is familiar to people with dementia, and the care recipient is highly receptive to it. A sensor or other device for controlling the gripping force can also be installed separately on the inside of the hand 63R.

Although not shown, a hand sensor 83L including a similar vital sensor to the hand sensor 83R is provided on the outside of the part 63BL of the hand 63L.

Hereafter, when there is no need to distinguish between the hand sensor 83L and the hand sensor 83R individually, the hand sensors will simply be referred to as hand sensors 83.

As shown in Figs. 10 to 12, the hand display 91 can be mounted to the hand 63L of the nursing care robot 11. For example, the hand display 91 is mounted to the outside of the part 63BL of the hand 63L. The hand display 91 has for example an 8-inch display part.

For example, as shown in Figs. 11 and 12, the nursing care robot 11 performs tracking control by controlling the movable shaft of the arm 27L so that the screen of the hand display 91 faces the care recipient's head. This makes it possible to move the hand display 91 to a position that is easy for the care recipient to see, regardless of the care recipient's sitting posture.

Although not shown, the hand display 91 can also be attached to the hand 63R of the nursing care robot 11.

For example, a handle-shaped grip may be provided on the back of the hand display 91, so that the nursing care robot 11 can hold the hand display 91 with its hand 63L or 63R.

As described above, the nursing care robot 11 possesses a realistic presence comparable to that of a two-year-old child, is suitably compact, and is sized so that a seated elderly person can easily look down upon it. The nursing care robot 11 has a large head-to-height ratio to evoke the cute form of a grandchild. The eyeball parts 41 are positioned at a height that makes it easy for the care recipient to make eye contact, so that the care recipient can easily recognize the gaze and images displayed on the eyeball parts 41. The nursing care robot 11 does not have a different degree of freedom axes or range of motion from a human, making care robot 11's movements natural and easy for the care recipient to understand.

The head 21 is positioned at a height that allows the head to overlook the surface of the typical table 12, and the shoulders 25 and arms 27 are designed to easily approach the edges of the typical table 12.

Therefore, the nursing care robot 11 is likely to be accepted by the care recipients without causing feelings of intimidation or fear. The nursing care robot 11 may also execute motions of Humanitude or execute basic applications for active monitoring robots.

### <Exemplary configuration of cart 71>

Figs. 13 to 16 shows exemplary configurations of the cart 71 of the base part 23 of the nursing care robot 11. Fig. 13 is a perspective view of the cart 71 as viewed from the diagonal rear right Fig. 14 is a perspective view of the cart 71 removed of a top plate 111 as viewed from the diagonal front right. Fig. 15 is a plan view of the cart 71 removed of the top plate 111. Fig. 16 is an external view of the cart 71 removed of the top plate 111 as viewed from the diagonal front left and from below.

The cart 71 includes the top plate 111, a base plate 112, front wheels 113F, rear wheels 113B, left wheels 113L, right wheels 113R, dampers 114F to 114R, and springs 115FL to 115BR.

The top plate 111 and base plate 112 are made of metal. The front wheel 113F, the rear wheel 113B, the left wheel 113L, and the right wheel 113R each include a caster and a resin (e.g., PTFE) wheel provided therein.

The front wheel 113F is provided at the front end of the base plate 112 so that the wheel rotates in the front-to-back direction of the nursing care robot 11. Above the resin wheel housing covering the front wheel 113F is a resin (e.g., sponge rubber) damper 114F that extends in the vertical direction. The wheel housing and damper 114F of the front wheel 113F may be integrally formed.

The rear wheel 113B is provided at the rear end of the base plate 112 so that the wheel rotates in the front-to-back direction of the nursing care robot 11. A resin damper (e.g., sponge rubber) 114B that extends in the up-down direction is provided above the wheel housing made of resin that covers the rear wheel 113B. The wheel housing and the damper 114B of the rear wheel 113B may be integrally formed.

The left wheel 113L is provided on the left end of the base plate 112 so that the wheel rotates in the front-to-back direction of the nursing care robot 11. A damper 114L made of resin (e.g., sponge rubber) is provided on the upper end of the resin wheel housing covering the left wheel 113L. The wheel housing and the damper 114L of the left wheel 113L may be integrally formed.

The right wheel 113R is provided on the right end of the base plate 112 so that the wheel rotates in the front-to-back direction of the nursing care robot 11. A resin damper 114R (e.g., sponge rubber) is provided on the upper end of the resin wheel housing covering the wheel of the right wheel 113R. The wheel housing and the damper 114R of the right wheel 113R may be integrally formed.

In the following, when it is not necessary to distinguish between the front wheel 113F, the rear wheel 113B, the left wheel 113L, and the right wheel 113R individually, they will be referred to as the wheels 113. Hereinafter, when it is not necessary to distinguish between the dampers 114F to 114R individually, they will simply be referred to as the dampers 114.

The front wheel 113F and the rear wheel 113B are passive wheels, while the left wheel 113L and the right wheel 113R are drive wheels. Each of the wheels 113 do not have a turning mechanism, and the nursing care robot 11 turns by differential movement between the left wheel 113L and the right wheel 113R. Since the wheels 113 are made of resin, vibration caused by the cart 71 slipping on the ground when the cart 71 turns is suppressed.

The springs 115FL and 115BR are arranged along the outer periphery of the base plate 112. Specifically, the spring 115FL is located between the front wheel 113F and the left wheel 113L, and is slightly closer to the front wheel 113F. The spring 115FR is located between the front wheel 113F and the right wheel 113R, and is slightly closer to the front wheel 113F. The spring 115BL is located almost in the middle between the rear wheel 113B and the left wheel 113L. The spring 115BR is located almost in the middle between the rear wheel 113B and the right wheel 113R.

Hereinafter, when it is not necessary to distinguish between the springs 115FL to 115BR individually, they will simply be referred to as springs 115.

In this way, each damper 114 and each spring 115 are arranged at a phase difference of about 45°. Each damper 114 and each spring 115 are arranged so that the stroke t of the spring 115 and the natural length d of the damper 114 are equal.

The top plate 111 is fixed to the cart 71 via the dampers 114 and the springs 115. The body of the nursing care robot 11 above the cart 71 is provided on the top surface of the top plate 111. In this way, the upper body of the nursing care robot 11 and the wheels 113 of the cart 71 are connected via the dampers 114 and springs 115 and thus structurally insulated from each other.

Then, the high-frequency (short-period) vibration of the wheels 113 is absorbed by the springs 115, and the high-frequency vibration of the upper body of the nursing care robot 11 is suppressed. The low-frequency (long-period) vibration of the wheels 113 is absorbed by the dampers 114, and the low-frequency vibration of the upper body of the nursing care robot 11 is suppressed. Therefore, the vibration of the wheels 113 of the nursing care robot 11 during translation and turning is absorbed by the dampers 114 and the springs 115, and the vibration of the upper body of the nursing care robot 11 is suppressed.

In this way, the quality of applications to be executed by the nursing care robot 11 is improved as the vibration of the upper body of the nursing care robot 11 is suppressed. For example, when the nursing care robot 11 executes an application that involves interaction with a care recipient, the upper body of the nursing care robot 11 can be prevented from swaying, thereby avoiding causing anxiety to the care recipient. For example, when the nursing care robot 11 executes an application for delivering items, the vibration of the items is restrained.

For example, this improves space efficiency when compared to the case where a suspension is fitted to each wheel 113, and the vibration of the upper body of the nursing care robot 11 can be restrained while making the size of the cart 71 more compact.

### <Modifications of the layout of dampers 114 and springs 115>

Fig. 17 shows modifications of the layout of the dampers 114 and springs 115 of the cart 71. In Fig. 17 at A to C, the upward direction indicates the front direction of the nursing care robot 11.

In the example shown in Fig. 17 at A, four springs 115 are arranged along the outer circumference of the base plate 112, at intervals of approximately 90° positioned at the front, back, left, and right Four dampers 114 are also arranged along the outer circumference of the base plate 112, at intervals of approximately 90° each about at the midpoint between adjacent springs 115.

In the example in Fig. 17 at B, three springs 115 are arranged along the outer circumference of the base plate 112, at intervals of approximately 120°. One of the three springs 115 is arranged in the front direction of the base plate 112. Three dampers 114 are arranged along the outer circumference of the base plate 112, at intervals of approximately 120° each about at the midpoint between adjacent springs 115.

In the example in Fig. 17 at C, as compared to the example in Fig. 17 at B, the positions of the dampers 114 and the spring 115 are reversed. In other words, the dampers 114 are arranged in the positions where the springs 115 are arranged in Fig. 17 at B, and the springs 115 are arranged in the positions where the dampers 114 are arranged in Fig. 17 at B.

### <Exemplary configuration of robot operation system 1>

Fig. 18 schematically shows an exemplary configuration of a robot operation system 201 that operates the nursing care robot 11 described above.

The robot operation system 201 includes the nursing care robot 11, a controller 211, a charging dock 212, a Wi-Fi router 213, and a facility LAN 214.

In addition to the eyeball displays 55L and 55R and the hand display 91 described above, the nursing care robot 11 also has a distance image sensor 231, a microphone 232, a vital sensor 233, a LiDAR 234, a robot internal PC (personal computer) 235, a robot internal PC 236, a head LED indicator 237, a speaker 238, an actuator 239, an emergency stop switch 240, a bumper sensor 241, a cart drive unit 242, an actuator 243, a power supply management unit 244, and a power supply 245.

The distance image sensor 231 is provided for example in the head sensor 81 of the nursing care robot 11. The distance image sensor 231 captures images of the surroundings of the nursing care robot 11 and supplies the distance image data obtained by the imaging to the robot internal PC 235. The distance image data indicates for example the captured images and the depth values (distances) of pixels.

The distance image sensor 231 may include multiple sensors. For example, the distance image sensor 231 may include two types of sensors: an image sensor and a distance sensor. In this case, for example, the image sensor may be provided in the head sensor 81, and the distance sensor may be provided in the head sensor 81 or the chest sensor 82.

The microphone 232 is, for example, provided in the head sensor 81 of the nursing care robot 11. The microphone 232 collects sounds around the nursing care robot 11 and supplies the sound data indicating the collected sounds to the robot internal PC 235.

The vital sensor 233 is provided for example in the chest sensor 82 and the hand sensor 83 of the nursing care robot 11. The vital sensor 233 includes one or more sensors that measure the value of the vital signs of the care recipient, such as body temperature, heart rate, SpO2, blood pressure, and respiratory rate. The vital sensor 233 supplies the vital data indicating the measurement result of the value of the vital signs to the robot internal PC 235.

For example, the LiDAR 234 is provided in the head sensor 81 of the nursing care robot 11. The LiDAR 234 detects the distance to each point around the nursing care robot 11, generates point cloud data indicating the detection result, and supplies the data to the robot internal PC 235.

The robot internal PC 235 includes for example a central processing unit (CPU), a read-only memory (ROM), a random access memory (RAM). The robot internal PC 235 executes control of various parts of the nursing care robot 11 and various types of processing. The robot internal PC 235 includes a sensor information processing unit 261, an application setting unit 262, an application control unit 263, and an action control unit 264.

The sensor information processing unit 261 performs processing of information detected by the various sensors of the nursing care robot 11. For example, the sensor information processing unit 261 evaluates the care recipient's condition and detects obstacles around the nursing care robot 11 on the basis of information detected by the various sensors. The sensor information processing unit 261 includes an image information processing unit 281, a sound information processing unit 282, a vital information processing unit 283, and a LiDAR information processing unit 284.

The image information processing unit 281 performs various kinds of image processing on the basis of the distance image data. For example, the image information processing unit 281 detects the position, shape, type, movement, etc. of objects around the nursing care robot 11. For example, the image information processing unit 281 performs processing such as authenticating people around the nursing care robot 11, detecting line of sight or facial orientation, detecting gestures, and recognizing facial expressions. The image information processing unit 281 supplies the information indicating the result of the image processing to the action control unit 264.

The sound information processing unit 282 performs various kinds of sound processing on the basis of sound data. For example, the sound information processing unit 282 performs sound recognition processing to recognize the content of the care recipient's speech. The sound information processing unit 282 supplies the information indicating the result of the sound processing to the action control unit 264.

The vital information processing unit 283 executes the processing of detecting the value of the vital signs of the care recipient on the basis of the vital data. The vital information processing unit 283 supplies the vital information indicating the detection result of the value of the vital signs to the action control unit 264.

The LiDAR information processing unit 284 performs the processing of detecting for example the position, shape, and movement of objects around the nursing care robot 11 on the basis of the point cloud data. The LiDAR information processing unit 284 supplies the information indicating the result of the processing of detecting for example the position, shape, and movement of objects around the nursing care robot 11 to the action control unit 264.

The application setting unit 262 sets various pieces of information for executing an application in response to an instruction from the controller 211. The application setting unit 262 includes a user setting data storage unit 301 and an application execution scheduler 302.

The user setting data storage unit 301 generates and stores individual operational parameters for each care recipient when executing an application in response to an instruction from the controller 211. The operational parameters are set on the basis of information about the receptiveness of each care recipient to the nursing care robot 11. Examples of the operational parameters include those related to whether to initiate talking, voice volume, speech rate, physical movement speed, travel speed, approachable distance, whether physical contact is permissible, hobbies and preferences, and other special items.

The application execution scheduler 302 generates and stores schedule information related to schedules for executing applications, in response to an instruction from the controller 211. The schedule information includes, for example, information about the date and time when an application is to be executed, the type of application to be executed, and the care recipient for whom the application is to be executed.

The application control unit 263 controls execution of applications by the nursing care robot 11 by controlling the action control unit 264 on the basis of the operational parameters stored in the user setting data storage unit 301 and the schedule information stored in the application execution scheduler 302. For example, the application control unit 263 controls the type of application to be executed by the nursing care robot 11, the timing of application execution, and the care recipient for whom the application is to be executed. For example, the application control unit 263 controls the application execution method by the nursing care robot 11 for example on the basis of the operational parameters, in order to increase the receptiveness of each care recipient to the application.

The application control unit 263 also updates the operational parameters stored in the user setting data storage unit 301 on the basis of the reactions of the care recipient during the execution of each application detected by the sensor information processing unit 261.

The action control unit 264 controls various parts of the nursing care robot 11 in order to make the nursing care robot 11 perform actions for example based on various applications. The action control unit 264 includes an application information storage unit 321, a motion set information storage unit 322, a map information storage unit 323, a scenario execution control unit 324, a tracking motion control unit 325, a motor drive control unit 326, a sound output control unit 327, a navigation control unit 328, an automatic charging control unit 329, and a call communication function control unit 330.

The application information storage unit 321 stores application information related to each application executed by the nursing care robot 11. The application information includes, for example, a scenario for executing the application. The scenario includes information for example about the content of speech motions, the order in which speech motions are executed, and branching conditions for the speech motions.

A speech motion indicates a combination of speech and motion performed by the nursing care robot 11. In other words, a speech motion indicates a combination of the content of speech (dialog) output by the nursing care robot 11 and the motion (motion) of the nursing care robot 11 during speaking.

When the nursing care robot 11 performs only speech without moving, the speech motion indicates only the speech performed by the nursing care robot 11. When the nursing care robot 11 performs only motion without speech, the speech motion indicates only the motion performed by the nursing care robot 11.

For example, the nursing care robot 11 executes each application by performing multiple speech motions in order according to the scenario. The nursing care robot 11 changes for example whether to perform speech motions and the order of execution according to the situation.

The motion set information storage unit 322 stores motion set information used to execute each speech motion indicated in the scenario for each application. The motion set information includes, for example, the content of lines spoken by the nursing care robot 11 or sound data in the speech motion, and motion data that indicates the movement of each part of the nursing care robot 11.

A map information storage unit 323 stores map information for a nursing facility, where the nursing care robot 11 is used. The map information includes, for example, information about the location of each care recipient's room or seat.

The scenario execution control unit 324 controls the execution of a scenario corresponding to an application to be executed on the basis of the processing result by the sensor information processing unit 261. For example, the scenario execution control unit 324 detects a trigger that serves as a branching condition for a scenario. For example, the scenario execution control unit 324 detects triggers such as people, the direction of the care recipient's face, the volume of the care recipient's speech, and the content of the care recipient's speech. The scenario execution control unit 324 controls whether to execute speech motions contained in the scenario, the execution order, repetition, pausing, and canceling for example on the basis of the trigger detection result.

The tracking motion control unit 325 controls the execution of tracking motions on the basis of the detection result of at least one of the care recipient's face and gaze direction obtained by the sensor information processing unit 261. The tracking motion refers to the processing of tracking for example the care recipient's face and gaze direction and turning the face of the nursing care robot 11 or the screen of the hand display 91 toward the care recipient's face.

The motor drive control unit 326 controls the actuator 239 that drives the joints of the nursing care robot 11, thereby driving the joints of the nursing care robot 11 and controlling the movement and posture of the nursing care robot 11.

The sound output control unit 327 controls sound output from the speaker 238, such as the speech sound of the nursing care robot 11 and music.

The speech sound of the nursing care robot 11 is set to the voice of a child (e.g., a child aged 4 to 8 years old), for example, according to the concept of the robot as an infant grandchild robot. However, people with hearing disabilities have difficulty in hearing high-frequency sounds included in the speech of children.

To address this, the sound output control unit 327 for example adjusts the frequency characteristic of the speech sound output to match the care recipient's hearing ability. For example, if the care recipient has difficulty in hearing the standard speech sound of the nursing care robot 11, the sound output control unit 327 amplifies the low frequency band (100 to 1000 Hz) that is easier for the elderly to hear, and attenuates the high frequency band (2000 Hz or more). This makes it easier for the care recipient to hear the speech sound of the nursing care robot 11.

Since the high-frequency components are retained without modulating the entire speech sound, the speech sound does not become too low-pitched, and the sense of unnaturalness can be reduced compared to standard speech sound.

For example, the sound output control unit 327 may be configured to correct the frequency characteristic of the speech sound according to user settings. For example, the sound output control unit 327 may also correct the frequency characteristic of the speech sound so that it is easier for people with hearing difficulties to hear it, for example when it is necessary to capture the attention of the care recipient or to ensure the content of speech is communicated.

The navigation control unit 328 controls the direction, speed, and path of movement of the nursing care robot 11 by controlling the cart drive unit 242 for example on the basis of map information stored in the map information storage unit 323.

The automatic charging control unit 329 controls the automatic charging of the power supply 245 on the basis of information indicating the state of the power supply 245 supplied by the power supply management unit 244. For example, upon determining that the power supply 245 needs to be charged, the automatic charging control unit 329 instructs the navigation control unit 328 to move the nursing care robot 11 to the charging dock 212. The automatic charging control unit 329 also controls the power supply management unit 244 to charge the power supply 245 after the nursing care robot 11 moves to the charging dock 212.

The call communication function control unit 330 controls the call and communication functions for example with other communication devices and servers through the Wi-Fi router 213 and the facility LAN 214. For example, the call communication function control unit 330 controls the video call function using the hand display 91.

For example, the robot internal PC 236 may be a computer including a CPU, a ROM, and a RAM. The robot internal PC 236 executes for example display control for the nursing care robot 11. The robot internal PC 236 includes a display control unit 351.

The display control unit 351 controls the display function of the nursing care robot 11. For example, the display control unit 351 controls the display of the black eye part 52L by the eyeball display 55L and the black eye part 52R by the eyeball display 55R. The display control unit 351 controls the blinking of the head LED indicator 237.

The head LED indicator 237 is provided on the head of the nursing care robot 11 and uses LEDs to indicate information for example about the state of the nursing care robot 11.

The speaker 238 outputs sound such as the speech sound of the nursing care robot 11 and music under the control of the sound output control unit 327.

The actuator 239 drives the joints of the nursing care robot 11 under the control of the motor drive control unit 326.

The emergency stop switch 240 is used to stop the nursing care robot 11 in an emergency. When the emergency stop switch 240 is operated, the emergency stop switch 240 supplies an operation signal to the cart drive unit 242.

The bumper sensor 241 is for example installed on the front of the cart 71 and performs processing such as detecting collisions with objects on the front of the cart 71 and supplies sensor data indicating the detection result to the cart drive unit 242.

The cart drive unit 242 controls the moving direction and speed of the nursing care robot 11 by controlling the actuator 243 of the cart 71 under the control of the action control unit 264. The cart drive unit 242 also stops the movement of the nursing care robot 11 by controlling the actuator 243 in response to an operation signal input from the emergency stop switch 240. Furthermore, the cart drive unit 242 controls the moving direction and speed of the nursing care robot 11 by controlling the actuator 243 according to the sensor data from the bumper sensor 241.

The actuator 243 drives the left and right wheels 113L and 113R of the cart 71 which are drive wheels under the control of the cart drive unit 242.

The power supply management unit 244 manages the power supply 245. For example, the power supply management unit 244 monitors the state of the power supply 245 (e.g., the remaining amount of power in the power supply 245), and supplies information indicating the state of the power supply 245 to the action control unit 264. For example, the power supply management unit 244 controls the charging of the power supply 245 using the charging dock 212.

The power supply 245 includes for example a battery and supplies power to various parts of the nursing care robot 11.

The controller 211 is a robot controller device that can be carried or worn by nursing home staff. The controller 211 is used for example to set operational parameters for each care recipient and schedule information for each application. Multiple controllers 211 may be provided, and multiple staff members may carry or wear controllers 211.

The controller 211 includes a joystick-type input device 371 and a tablet-type terminal device 372.

The joystick-type input device 371 is used for example to set operational parameters for each care recipient and schedule information for each application.

The tablet-type terminal device 372 includes a display, an operation panel, and a communication device. The tablet-type terminal device 372 is used, for example, to set operational parameters for each care recipient and schedule information for each application. The tablet-type terminal device 372 performs communication with the nursing care robot 11.

The charging dock 212 is located in the nursing care facility and is used for standby charging of the power supply 245 of the nursing care robot 11. Multiple charging docks 212 may be provided in the nursing care facility.

The Wi-Fi router 213 is located in the nursing care facility and used for the nursing care robot 11 to communicate with the outside. Multiple Wi-Fi routers 213 may be provided in the nursing care facility.

The facility LAN 214 is located in the nursing care facility and connected to various communication devices and information processing devices.

### <Processing by nursing care robot 11>

Next, the processing by the nursing care robot 11 will be described with reference to Figs. 19 to 36.

In the following description, the processing when the nursing care robot 11 executes an application for the care recipient 14 sitting in the chair 13 in front of the table 12, as shown in Fig. 19, will be described.

### <Basic flow of application execution processing>

First, referring to the flowchart in Fig. 20, the basic flow of the application execution processing executed by the nursing care robot 11 will be described.

The processing starts when it is time to execute an application to perform prescribed care for a care recipient 14 and the application control unit 263 gives a command to the action control unit 264 to execute the application.

For example, the nursing care robot 11 executes an application to perform care for the care recipient 14 by carrying out the steps of preparing for meeting, preparing for care, connecting perception, performing emotional fixation, and making a promise to meet again in order using Humanitude.

Specifically, in step S1, the nursing care robot 11 greets from a distance and executes a preparation for meeting.

For example, the navigation control unit 328 controls the cart drive unit 242 to move the nursing care robot 11 from the initial position P0 to a long-distance position P3, as shown in Fig. 19. The long-distance position P3 is set for example to a direction of about 0° relative to the front of the care recipient 14 and a position about 2 m away from the care recipient 14.

At the long-distance position P3, the nursing care robot 11 greets the care recipient 14. Specifically, for example, the motor drive control unit 326 controls the actuator 239 under the control of the tracking motion control unit 325 to direct the face of the nursing care robot 11 toward the face of the care recipient 14. The motor drive control unit 326 controls the actuator 239 to make the nursing care robot 11 perform a motion for greeting the care recipient 14. The sound output control unit 327 outputs speech sound corresponding to the greeting from the speaker 238 according to the motion of the nursing care robot 11.

In step S2, the nursing care robot 11 moves close to greet the care recipient 14, and starts the main scenario part of the application.

For example, the navigation control unit 328 controls the cart drive unit 242 to move the nursing care robot 11 from the long-distance position P3 to the medium-distance position P2, as shown in Fig. 19. The medium-distance position P2 is set for example in a direction at about 45° with respect to the front of the care recipient 14 and at a position about 1.2 m away from the care recipient 14. Then, the nursing care robot 11 greets the care recipient 14 at the medium-distance position P2 using the same processing as the step S1.

Then, the navigation control unit 328 controls the cart drive unit 242 as required to move the nursing care robot 11 from the medium-distance position P2 to the short-distance position P1, as shown in Fig. 19. The short-distance position P1 is set for example in a direction at about 70° with respect to the front of the care recipient 14 and a position about 0.6 m away from the care recipient 14. Then, the nursing care robot 11 greets the care recipient 14 at the short-distance position P1 using the same processing as the step S1.

The medium-distance position P2 and the short-distance position P1 are desirably in the direction of the dominant hand of the care recipient 14. In this case, the nursing care robot 11 approaches the care recipient 14 so that, for example, the angle with respect to the front of the care recipient 14 increases toward the dominant hand as the robot approaches the care recipient 14.

When there are no obstacles directly in front of the care recipient 14, the long-distance position P3, the medium-distance position P2, and the short-distance position P1 may all be set to a direction at approximately 0° with respect to the front of the care recipient 14.

In this way, physical Humanitude is performed to the care recipient 14. In other words, an approach that stepwise reduces the physical distance between the nursing care robot 11 and the care recipient 14 is executed. This allows the nursing care robot 11 to approach the care recipient 14 without causing negative emotions (e.g., fear), while giving a sense of security, which improves the receptiveness of the care recipient 14 to the nursing care robot 11.

Then, the nursing care robot 11 starts the main scenario part of the application under the control of the scenario execution control unit 324.

In step S3, the nursing care robot 11 starts presenting image information using the hand display 91. For example, the scenario execution control unit 324 displays image information related to the content to be provided by the application on the hand display 91.

In step S4, the nursing care robot 11 executes reality orientation. Here, the reality orientation refers to processing that conveys orientation information related to the content to be provided by the application being executed to the care recipient 14.

For example, the motor drive control unit 326 controls the actuator 239 under the control of the scenario execution control unit 324 to make the nursing care robot 11 perform motions to convey the orientation information related to the content to the care recipient 14. The sound output control unit 327 outputs sound from the speaker 238 to convey the orientation information related to the content to the care recipient 14 under the control of the scenario execution control unit 324.

In step S5, the nursing care robot 11 executes cognitive stimulation therapy. For example, the nursing care robot 11 performs cognitive stimulation therapy by asking the care recipient 14 questions related to the content executed by the application in the process of execution.

For example, the motor drive control unit 326 controls the actuator 239 under the control of the scenario execution control unit 324, causing the nursing care robot 11 to perform motions to ask the care recipient 14 questions related to the content. The sound output control unit 327 has sound output from the speaker 238 to ask the care recipient 14 questions related to the content, under the control of the scenario execution control unit 324.

In step S6, the nursing care robot 11 introduces and explains the content. For example, the nursing care robot 11 communicates information directly representing the content provided by the application in the process of execution (e.g., the title of the content or its main content, etc.) to the care recipient 14.

For example, the motor drive control unit 326 controls the actuator 239 under the control of the scenario execution control unit 324 to make the nursing care robot 11 perform a motion to convey the information directly representing the content to the care recipient 14. The sound output control unit 327 outputs sound from the speaker 238 to convey the information that directly represents the content to the care recipient 14 under the control of the scenario execution control unit 324.

Through the processing in steps S2 to S6, the preparation for care in Humanitude is executed. In other words, the care recipient 14 is guided to the content by the nursing care robot 11 that presents the information related to the content.

In step S7, the nursing care robot 11 provides the content. For example, the motor drive control unit 326 controls the actuator 239 under the control of the scenario execution control unit 324 to make the nursing care robot 11 perform motions corresponding to the content. The sound output control unit 327 outputs sound corresponding to the content from the speaker 238 under the control of the scenario execution control unit 324.

Through the processing in step S7, for example the perception connection in Humanitude is executed.

In this way, cognitive Humanitude is performed to the care recipient 14. In other words, an approach that stepwise reduces the cognitive distance of the care recipient 14 to the content provided by the application is performed. As a result, the nursing care robot 11 can provide the content while giving the care recipient 14 a sense of security without causing the care recipient to feel negative emotions (e.g., fear). As a result, the receptiveness of the care recipient 14 to the provided content improves.

In step S8, the nursing care robot 11 executes emotional fixation. For example, the motor drive control unit 326 controls the actuator 239 under the control of the scenario execution control unit 324, causing the nursing care robot 11 to perform motions to give the care recipient 14 positive emotions while reviewing the provided content with the care recipient 14. The sound output control unit 327, under the control of the scenario execution control unit 324, outputs sound from the speaker 238 to give the care recipient 14 positive emotions while reviewing the provided content with the care recipient 14.

If the currently running application contains multiple contents, for example, the processing in steps S3 to S7 or the processing in steps S3 to S8 is repeatedly executed.

The processing in all steps S3 to S6 does not have to be executed. For example, at least one of these is executed according to the characteristic of the content to be provided and the characteristic of the care recipient 14.

In step S9, the nursing care robot 11 executes the promise to meet again. For example, the motor drive control unit 326 controls the actuator 239 under the control of the scenario execution control unit 324 to communicate the next appointment to the care recipient 14 and to have the nursing care robot 11 execute a motion to say goodbye. The sound output control unit 327, under the control of the scenario execution control unit 324, communicates the next appointment to the care recipient 14 and outputs sound from the speaker 238 to say goodbye. The navigation control unit 328 controls the cart drive unit 242 to move the nursing care robot 11 away from the care recipient 14.

Then, the application execution processing ends.

### <First specific example of application execution processing>

Next, referring to the flowchart in Fig. 21, a first specific example of application execution processing will be described.

In the first specific example of application execution processing, the main content of the application is provided at the medium-distance position P2.

In step S101, the nursing care robot 11 moves to the long-distance position P3. For example, the navigation control unit 328 controls the cart drive unit 242 to move the nursing care robot 11 from the initial position P0 to the long-distance position P3, as shown in Fig. 19.

In step S102, the nursing care robot 11 executes a speech motion. For example, the motor drive control unit 326 controls the actuator 239 under the control of the scenario execution control unit 324 to make the nursing care robot 11 perform a scenario-based motion. The sound output control unit 327 outputs scenario-based speech sound from the speaker 238 under the control of the scenario execution control unit 324.

In step S103, the nursing care robot 11 determines whether the gaze of the care recipient 14 has been detected. Specifically, the image information processing unit 281 detects at least one of the face and gaze direction of the care recipient 14 on the basis of distance image sensor data from the distance image sensor 231 and supplies the information indicating the detection result to the action control unit 264.

If the face or line of sight of the care recipient 14 does not face toward the nursing care robot 11, the scenario execution control unit 324 determines that the gaze of the care recipient 14 has not been detected, and the process proceeds to step S104.

In step S104, the scenario execution control unit 324 determines whether the state without detection of the gaze of the care recipient 14 has continued for at least n seconds. If it is determined that the state without detection of the gaze of the care recipient 14 has not continued for at least n seconds, the process returns to step S102.

Thereafter, the processing in steps S102 to S104 is repeated until it is determined in step S103 that the gaze of the care recipient 14 has been detected, or it is determined in step S104 that the state without detection of the gaze of the care recipient 14 has continued for at least n seconds. More specifically, the speech motion in step S102 is repeatedly executed.

Meanwhile, in step S103, the scenario execution control unit 324 determines that the gaze of the care recipient 14 has been detected if the face or the line of sight of the care recipient 14 is directed toward the nursing care robot 11, and the process proceeds to step S105.

In step S105, the nursing care robot 11 moves to the medium-distance position P2. For example, the navigation control unit 328 controls the cart drive unit 242 to move the nursing care robot 11 from the long-distance position P3 to the medium-distance position P2, as shown in Fig. 19.

In step S106, the next speech motion is executed by the same processing as in step S102. The speech motion executed in step S106 and the speech motion executed in step S102 are usually different speech motions.

In step S107, the nursing care robot 11 executes branching dialog motion processing.

Here, referring to the flowchart in Fig. 22, details of the branching dialog motion processing will be described.

In step S151, similarly to the processing in step S103 in Fig. 21, it is determined whether the gaze of the care recipient 14 has been detected. If it is determined that the gaze of the care recipient 14 has not been detected, the process proceeds to step S152.

In step S152, the nursing care robot 11 changes the sound and executes a speech motion again. Specifically, the nursing care robot 11 performs again at least a part of the speech motion performed in step S106 in Fig. 21. At the time, for example, the sound output control unit 327 increases the volume of the speech sound output from the speaker 238 and emphasizes the low frequency range. This is expected to make it easier for the care recipient 14 to hear the speech sound output from the nursing care robot 11 and to pay attention to the speech motion of the nursing care robot 11.

In step S153, it is determined whether the gaze of the care recipient 14 has been detected, similarly to the processing in step S103 in Fig. 21. If it is determined that the gaze of the care recipient 14 has not been detected, the process proceeds to step S154.

In step S154, the nursing care robot 11 moves to a position within the care recipient's field of vision and executes the speech motion again. Specifically, the tracking motion control unit 325 controls the cart drive unit 242 on the basis of the direction of the face or line of sight of the care recipient 14 and moves the nursing care robot 11 to a position within the field of vision of the care recipient 14. The nursing care robot 11 then performs again at least a part of the speech motion executed in step S106 in Fig. 21.

In step S155, it is determined whether the gaze of the care recipient 14 has been detected, similarly to the processing in the step S103 in Fig. 21. If it is determined that the gaze of the care recipient 14 has not been detected, the process proceeds to step S156.

In step S156, the nursing care robot 11 raises and moves its hand within the field of vision, and then executes the speech motion again. For example, the tracking motion control unit 325 controls the actuator 239 to raise the hand 63 that does not hold the hand display 91 to the height of the head 21 and to perform an action such as waving the hand within the field of vision of the care recipient 14. The nursing care robot 11 performs again at least a part of the speech motion executed in step S106 in Fig. 21.

In step S157, it is determined whether the gaze of the care recipient 14 has been detected, similarly to the processing in step S103 in Fig. 21. If it is determined that the gaze of the care recipient 14 has not been detected, the process proceeds to step S158.

In step S158, the nursing care robot 11 knocks on the table 12 of the care recipient 14 and executes the speech motion again. For example, the tracking motion control unit 325 controls the actuator 239 to make the hand 63 that does not hold the hand display 91 knock the tabletop 12A of the table 12. The nursing care robot 11 then performs again at least a part of the speech motion performed in step S106 in Fig. 21.

In step S159, it is determined whether the gaze of the care recipient 14 has been detected, similarly to the processing in step S103 Fig. 21. If it is determined that the gaze of the care recipient 14 has not been detected, the process proceeds to step S160.

In step S160, the nursing care robot 11 touches the arm of the care recipient 14 and executes the speech motion again. For example, the tracking motion control unit 325 controls the actuator 239 to make the hand 63 that does not hold the hand display 91 touch the arm of the care recipient 14. The nursing care robot 11 executes again at least a part of the speech motion executed in step S106 in Fig. 21.

In step S161, it is determined whether the gaze of the care recipient 14 has been detected, similarly to the processing in step S103 in Fig. 21. If it is determined that the gaze of the care recipient 14 has not been detected, the process proceeds to step S162.

In step S162, it is determined whether the state without detection of the gaze of the care recipient 14 has continued for at least n seconds, similarly to the processing in step S104 in Fig. 21. If it is determined that the state without detection of the gaze of the care recipient 14 has not continued for at least n seconds, the process returns to step S161.

The processing in steps S161 and S162 is then repeated until it is determined in step S161 that the gaze of the care recipient 14 has been detected, or until it is determined in step S162 that the state without detection of the gaze of the care recipient 14 has continued for at least n seconds.

Meanwhile, if it is determined that the gaze of the care recipient 14 has been detected in steps S151, S153, S155, S157, S159, or S161, the process proceeds to step S163.

In step S163, the sound information processing unit 282 determines whether speech by the care recipient 14 has been detected on the basis of sound data supplied by the microphone 232. If it is determined that the speech of the care recipient 14 has been detected, the process proceeds to step S164.

In step S164, the sound information processing unit 282 recognizes the content of the speech. For example, the sound information processing unit 282 recognizes a phrase registered in advance from the speech of the detected care recipient 14. The registered phrase may be a single word, or a combination of multiple words.

In step S165, the nursing care robot 11 executes a response motion according to the content of the speech. Specifically, the sound information processing unit 282 notifies the action control unit 264 of the recognition result of the content of the speech. In other words, the sound information processing unit 282 notifies the action control unit 264 of the phrase recognized from the speech of the care recipient 14.

The scenario execution control unit 324 selects the type of response to the speech of the care recipient 14 according to the recognition result of the speech content. For example, the scenario execution control unit 324 selects the type of response to be performed to on the basis of the phrase in the speech recognized by the sound information processing unit 282.

For example, the responses are classified under the following ten types.
1. Gratitude for information
2. Reply
3. Affirmation
4. Consideration
5. Waiting
6. Praise
7. Empathy
8. Greetings
9. Appreciation
10. Name

For example, the responses classified under the "1. Gratitude for information" include "Thank you for letting me know about it."

For example, the responses classified under "2. Reply" include "Yes" and "Yeah."

For example, the responses classified under "3. Affirmation" include "Uh-huh" and "I see."

For example, the responses classified under "4. Consideration" include "Are you okay?" "I understand," and "I will let the staff know about it."

For example, the responses classified under "5. Waiting" include "It won't be long" and "Just a moment please."

For example, the responses classified under "6. Praise" include "You look nice today as always," "Well done," and "Wonderful as expected."

For example, the responses classified under "7. Empathy" include "Good," and "Happy for you."

For example, the responses classified under "8. Greetings" include "Good morning," and "Hello."

The responses classified under "9. Appreciation" include "Thank you."

The responses classified under "10. Name" include "I am Hanamo" and "Call me Hana-chan." "Hanamo" is an example of the name of the nursing care robot 11.

For example, when the care recipient 14 says "My back hurts," the sound information processing unit 282 recognizes the phrase "hurts." In response to this, the scenario execution control unit 324 selects a response such as "Are you okay?" "Thank you for letting me know about it," and "I'll let the staff know about it."

For example, when the care recipient 14 says "I want to go home," the sound information processing unit 282 recognizes the phrase "want to go home" or "go home." In response to this, the scenario execution control unit 324 selects a response such as "Are you okay?" "Thank you for letting me know about it," and "I'll let the staff know about it."

For example, when the care recipient 14 says "I couldn't sleep again last night," the sound information processing unit 282 recognizes the phrase "I couldn't sleep." In response to this, the scenario execution control unit 324 selects a response such as "I understand," "Are you okay?" and "I'll let the staff know about it."

For example, if the care recipient 14 says "Is the meal ready yet?" the sound information processing unit 282 recognizes the phrase "meal ready yet." In response to this, the scenario execution control unit 324 selects a response such as "It won't be long, so please wait" or "Thank you for letting me know about it."

For example, when the care recipient 14 says "Is the snack ready yet?" the sound information processing unit 282 recognizes the phrase "snack ready yet?" In response to this, the scenario execution control unit 324 selects a response such as "It won't be long, so please wait," and "Thank you for letting me know about it."

For example, when the care recipient 14 says "I'm thirsty," the sound information processing unit 282 recognizes the phrase "thirsty." In response to this, the scenario execution control unit 324 selects a response such as "Are you okay?" "It won't be long, so please wait," and "Thank you for letting me know about it."

For example, when the care recipient 14 says "Is the tea ready yet?" the sound information processing unit 282 recognizes the phrase "tea ready yet?" In response to this, the scenario execution control unit 324 selects a response such as "Really?" "It won't be long, so please wait," and "Thank you for letting me know."

For example, if the care recipient 14 says "What is the meal?" the sound information processing unit 282 recognizes the phrase "What is the meal?" In response to this, the scenario execution control unit 324 selects a response such as "Really?" "It won't be long, so please wait," and "Thank you for letting me know about it."

For example, when the care recipient 14 says "What is the snack?" the sound information processing unit 282 recognizes the phrase "What is the snack?" In response to this, the scenario execution control unit 324 selects a response such as "Really?" "It won't be long, so please wait," and "Thank you for letting me know about it."

For example, when the care recipient 14 says "What is your name?" the sound information processing unit 282 recognizes the phrase "your name?" In response to this, the scenario execution control unit 324 selects a response such as "It's Hanamo" and "Please call me Hana-chan."

For example, when the care recipient 14 says "Who is this, may I ask?" the sound information processing unit 282 recognizes the phrase "Who is this." In response to this, the scenario execution control unit 324 selects a response such as "It's Hanamo" and "Call me Hana-chan."

For example, when the care recipient 14 says "How are you?" the sound information processing unit 282 recognizes the phrase "How are you?" In response to this, the scenario execution control unit 324 will select an appropriate response such as "Yes!" and "Hello.".

For example, when the care recipient 14 says "I was born in Taisho era," the sound information processing unit 282 recognizes the phrase "born in Taisho." In response to this, the scenario execution control unit 324 selects, responses such as "Uh-huh," "Wow," and "Thank you for letting me know about it."

For example, when the care recipient 14 says "I drew this picture," the sound information processing unit 282 recognizes the phrase "drew picture." In response to this, the scenario execution control unit 324 selects responses, for example, "Wow," "That's great," or "Thank you for letting me know about it."

For example, when the care recipient 14 says, "It's a picture of my grandson," the sound information processing unit 282 recognizes the phrase "a picture of my grandson." In response to this, the scenario execution control unit 324 selects a response, for example, "Uh-huh," "That's nice," or "Thank you for letting me know about it."

For example, when the care recipient 14 says "Thank you," the sound information processing unit 282 recognizes the phrase "Thank you." In response to this, the scenario execution control unit 324 selects a response such as "Thank you."

For example, when the care recipient 14 says, "Thank you for taking care of me," the sound information processing unit 282 recognizes the phrase "taking care of me." In response to this, the scenario execution control unit 324 selects an affable phrase such as, for example, "Thank you for telling me."

For example, when the care recipient 14 says "Good morning," the sound information processing unit 282 recognizes the phrase "Good morning." In response, the scenario execution control unit 324 selects a response such as "Good morning."

For example, when the care recipient 14 says "Hello," the sound information processing unit 282 recognizes the phrase "Hello." In response, the scenario execution control unit 324 selects, for example, a response such as "Hello."

For example, when the care recipient 14 says "I slept well," the sound information processing unit 282 recognizes the phrase "slept well" or "slept." In response, the scenario execution control unit 324 selects a response, for example, "Really?" "Um-hum," or "Thank you for letting me know about it."

For example, when the care recipient 14 says "The food was delicious," the sound information processing unit 282 recognizes the phrase "was delicious" or "delicious." In response to this, the scenario execution control unit 324 selects, a response, for example, "Really?" "Good for you," or "Thank you for letting me know about it."

For example, when the care recipient 14 says, "My back doesn't hurt today," the sound information processing unit 282 recognizes the phrase "doesn't hurt." In response to this, the scenario execution control unit 324 selects a response, for example, "Really?" "Good for you," or "Thank you for letting me know about it."

When no relevant phrase is recognized from the speech of the care recipient 14, or when the content of the speech cannot be recognized, the scenario execution control unit 324 selects a response belonging to "3. Affirmation." For example, when the care recipient 14 says "I like cats," and the sound information processing unit 282 fails to recognize any corresponding phrase, the scenario execution control unit 324 selects, for example, "uh-huh," or a similar response.

The nursing care robot 11 then executes a response motion including the selected response. Specifically, the sound output control unit 327 causes the speech sound of the selected response to be output from the speaker 238. The motor drive control unit 326 also controls the actuator 239 to execute a motion corresponding to the response as required.

Thereafter, the process returns to step S151, and the processing in steps S151 to S165 is repeated until it is determined in step S162 that the state without detection of the gaze of the care recipient 14 has continued for at least n seconds, or until it is determined in step S163 that speech from the care recipient 14 has not been detected.

Meanwhile, if it is determined in step S162 that the state without detection of the gaze of the care recipient 14 has continued for at least n seconds, or if it is determined in step S163 that speech from the care recipient 14 has not been detected, the branching dialog motion processing ends.

In this way, in the branching dialog motion processing, at least a part of the speech motion is performed again by the nursing care robot 11, while adding a motion to encourage the care recipient 14 to gaze and changing the sound. This encourages the care recipient 14 to gaze at the nursing care robot 11.

Referring back to Fig. 21, in step S108, the scenario execution control unit 324 determines, on the basis of the result of the processing in step S107, whether the state without detection of the gaze of the care recipient 14 has continued for at least n seconds. If it is determined that the state without detection of the gaze of the care recipient 14 has not continued for at least n seconds, the process proceeds to step S109.

In step S109, the scenario execution control unit 324 determines whether there is the next speech motion. If it is determined that there is the next speech motion, the process returns to step S106.

Thereafter, the processing in steps S106 to S109 is repeated until it is determined in step S108 that the state without detection of the gaze of the care recipient 14 has continued for at least n seconds, or until it is determined in step S109 that there is no next speech motion. In this way, the speech motions of the application are executed sequentially according to the scenario.

Meanwhile, if it is determined in step S109 that there is no next speech motion, the process proceeds to step S110. This is the case when all the speech motions of the application have been executed according to the scenario.

If it is determined in step S108 that the state without detection of the gaze of the care recipient 14 has continued for at least n seconds, the processing in step S109 is skipped, and the process proceeds to step S110. This is the case, for example, when the application ends in the middle of the scenario because no positive response is obtained from the care recipient 14.

In step S110, the nursing care robot 11 performs the ending motion. For example, the nursing care robot 11 executes a speech motion corresponding to the promise to meet again as an ending motion, similarly to the processing in step S9 in Fig. 20.

Then, the application execution processing ends.

Meanwhile, if it is determined in step S104 that the state without detection of the gaze of the care recipient 14 has continued for at least n seconds, the application execution processing ends. This is the case, for example, when the application execution is discontinued before the main content of the application is executed because no positive response is obtained from the care recipient 14.

### <Second specific example of application execution processing>

Next, a second specific example of application execution processing will be described with reference to the flowchart in Fig. 23.

In the second specific example of application execution processing, the main content is provided at the medium-distance position P2 and the short-distance position P1.

In steps S201 to S207, the same processing as that in steps S101 to S107 in Fig. 21 is performed.

In step S208, similarly to the processing in step S108 in Fig. 21, it is determined whether the state without detection of the gaze of the care recipient 14 has continued for at least n seconds. If it is determined that the state without detection of the gaze of the care recipient 14 has continued for at least n seconds, the process proceeds to step S209.

In step S209, similarly to the processing in step S109 in Fig. 21, it is determined whether there is the next speech motion. If it is determined that there is the next speech motion, the process returns to step S206.

Thereafter, the processing in steps S206 to S209 is repeated until it is determined in step S208 that the state without detection of the gaze of the care recipient 14 has continued for at least n seconds, or until it is determined in step S209 that there is no next speech motion.

Meanwhile, if it is determined in step S209 that there is no next speech motion, the process proceeds to step S210. This is the case when all the speech motions at the medium-distance position P2 have been executed according to the scenario.

In step S210, the nursing care robot 11 moves to the short-distance position P1. For example, the navigation control unit 328 controls the cart drive unit 242 to move the nursing care robot 11 from the medium-distance position P2 to the short-distance position P1, as shown in Fig. 19.

In step S211, the next speech motion is performed, similarly to the processing in step S106 in Fig. 21.

In step S212, the branching dialog motion processing is executed similarly to the processing in step S107 in Fig. 21.

In step S213, similarly to the processing in step S108 in Fig. 21, on the basis of the result of the processing in step S212, it is determined whether the state without detection of the gaze of the care recipient 14 has continued for at least n seconds. If it is determined that the state without detection of the gaze of the care recipient 14 has not continued for at least n seconds, the process proceeds to step S214.

In step S214, similarly to the processing in step S109 in Fig. 19, it is determined whether there is the next speech motion. If it is determined that there is the next speech motion, the process returns to step S211.

Thereafter, the processing in steps S211 to S214 is repeated until it is determined in step S213 that the state without detection of the gaze of the care recipient 14 has continued for at least n seconds, or it is determined in step S214 that there is no next speech motion.

Meanwhile, if it is determined that there is no next speech motion in step S214, the process proceeds to step S215. This is the case when all the speech motions of the application have been executed according to the scenario.

If it is determined in step S213 that the state without detection of the gaze of the care recipient 14 has continued for at least n seconds, the processing in step S214 is skipped, and the process proceeds to step S215. This is the case, for example, when the application ends in the middle of the scenario because no positive response is obtained from the care recipient 14.

Furthermore, if it is determined in step S205 that the state without detection of the gaze of the care recipient 14 has continued for at least n seconds, the processing in steps S209 to S214 is skipped, and the process proceeds to step S215. This is the case, for example, when the application ends in the middle of the scenario because no positive response is obtained from the care recipient 14.

In step S215, an ending motion is performed, similarly to the processing in step S110 in Fig. 21.

### Thereafter, the application execution processing ends.

Meanwhile, if it is determined in step S204 that the state without detection of the gaze of the care recipient 14 has continued for at least n seconds, the application execution processing ends. This is the case, for example, when the application execution is discontinued before the main content of the application is executed because no positive response is obtained from the care recipient 14.

For example, after moving to the short-distance position P1, the nursing care robot 11 may move between the short-distance position P1 and the medium-distance position P2, if necessary.

### <Specific examples of applications>

Next, specific examples of applications executed by the nursing care robot 11 will be described. In particular, specific examples of scenarios executed in the applications will be described.

### <Vital measurement application>

First, with reference to Figs. 24 to 27, a specific example of a vital measurement application that measures the value of the vital signs such as the body temperature of the care recipient 14 will be described.

To start with, as shown in Fig. 24, the nursing care robot 11 moves to the long-distance position P3 and executes the preparation for meeting by performing a speech motion that includes the following lines.

### "Good morning."

### "Grandma! I'm coming to you now."

In response to this, when the care recipient 14 does not gaze at the nursing care robot 11, the nursing care robot 11 repeatedly executes this speech motion. Nevertheless, if the care recipient 14 does not gaze at the nursing care robot 11, the nursing care robot 11 ends the vital measurement application and moves away from the care recipient 14.

Meanwhile, if the care recipient 14 gazes at the nursing care robot 11, the nursing care robot 11 moves to the medium-distance position P2 as shown in Fig. 25 and begins the preparation for care by executing a speech motion that includes the following lines.

### "Grandma."

### "Grandma!"

### "I came to see you, Grandma."

In response to this, if the care recipient 14 does not gaze at the nursing care robot 11, the nursing care robot 11 executes the branching dialog motion processing described above with reference to Fig. 22, so that the care recipient 14 gazes at the nursing care robot 11. Nevertheless, if the care recipient 14 still does not gaze at the nursing care robot 11, the nursing care robot 11 executes an ending motion, ends the vital measurement application, and moves away from the care recipient 14.

Meanwhile, if the care recipient 14 gazes at the nursing care robot 11, the nursing care robot 11 performs a response motion according to the speech of the care recipient 14, if any. Then, at the timing when the care recipient 14 gazes at the nursing care robot 11 without speaking, the nursing care robot 11 executes a speech motion that includes the following lines.

Furthermore, although the description will not be repeated, it should be assumed that the series of processing is executed between each speech motion. In other words, if the branching dialog motion processing or the response motion is executed as required, and the care recipient 14 does not gaze at the nursing care robot 11, the application ends after the ending motion. Meanwhile, the next speech motion is executed when the care recipient 14 gazes at the nursing care robot 11 without speaking.

"I'm glad you're laughing, Grandma."

"I'm so glad you're looking good."

"I'm glad you're doing well."

"We all care about you, Grandma."

"Being healthy is the best, isn't it?"

Next, the nursing care robot 11 executes a speech motion that includes the following lines.

"It's time to take today's temperature."

"Temperature?"

"Your temperature."

"Shall I take your temperature now?"

Next, the nursing care robot 11 executes a speech motion that includes the following lines to complete the preparation for care.

"Temperature, it's time to take your temperature."

"You will be safe if you take your temperature."

"Now your temperature, shall I take your temperature?"

Next, the nursing care robot 11 moves to the short-distance position P1, as shown in Fig. 26, and performs perception connection by executing a speech motion that includes the following lines.

"I'm going to take your temperature now."

"Just look at me for a second."

"Beep (sound of the thermometer)."

"It was 36.2°C."

"I'm done taking your temperature."

"Are you feeling the same as usual?"

Next, the nursing care robot 11 performs emotional fixation by performing a speech motion that includes the following lines.

"The time for taking your temperature is now over."

"That was a lot of fun, wasn't it?"

"Thank you."

Next, the nursing care robot 11 moves to the medium-distance position P2, as shown in Fig. 27, and begins an appointment to meet again by performing a speech motion that includes the following lines.

"I'll be back to talk to you next at 2:00 p.m."

"I'm going to go get a meal ready, so wait for me."

"See you later."

Next, the nursing care robot 11 performs an ending motion, which is a speech motion that includes the following lines, to end the appointment to meet again.

"I'll be back."

"Wait for me."

The nursing care robot 11 then ends the vital measurement application and leaves the care recipient 14.

In this way, the nursing care robot 11 can perform vital measurements on the care recipient 14 while increasing the receptiveness of the care recipient 14 to the nursing care robot 11 and vital measurements through the use of Humanitude.

### <Music therapy application>

Next, with reference for example to Figs. 28 to 30, a specific example of a music therapy application in which the nursing care robot 11 sings a song with the care recipient 14 will be described.

First, as shown in Fig. 24, the nursing care robot 11 moves to the long-distance position P3 and executes preparation for the encounter by performing a speech motion that includes the following lines.

"Grandma."

"Good morning."

"Grandma! I'm coming to you now."

In response, if the care recipient 14 does not gaze at the nursing care robot 11, the nursing care robot 11 repeatedly performs the speech motion. If the care recipient 14 still does not gaze at the nursing care robot 11, the nursing care robot 11 then discontinues the music therapy application and leaves the care recipient 14.

Meanwhile, if the care recipient 14 gazes at the nursing care robot 11, the nursing care robot 11 moves to the medium-distance position P2 as shown in Fig. 25 and begins preparation for care by executing a speech motion that includes the following lines.

"Grandma."

"Grandma!"

"I've come to see you, Grandma."

Next, the nursing care robot 11 executes a speech motion that includes the following lines.

"I'm glad you're laughing, Grandma."

"It's another fine day today, isn't it?"

Next, the nursing care robot 11 executes a speech motion that includes the following lines.

"I got a picture of you, Grandma."

"Look at this."

"Look over here."

Next, the nursing care robot 11 lifts the hand display 91 up to near the head 21, as shown in Fig. 28. The nursing care robot 11 then initiates reality orientation for example by having the image in Fig. 29 at A displayed on the hand display 91. The image in Fig. 29 at A represents the theme of December and, for example, displays bells, a Christmas tree, and a snowman. In other words, the nursing care robot 11 begins the processing of communicating to the care recipient 14 the orientation concerning for example the season, date, and time related to a song about to be sung.

Next, the nursing care robot 11 executes a speech motion that includes the following lines.

"It's December, the season of winter, isn't it?"

"Christmas is coming."

"Only a few more sleeps until New Year's Day."

"There are so many fun things to do."

Next, the nursing care robot 11 executes a speech motion that includes the following lines and ends the reality orientation.

"I'm here to sing a song today."

"I'm going to sing a song."

"Sing with me."

Next, the nursing care robot 11 has the hand display 91 display the image in Fig. 29 at B. The image in Fig. 29 at B is an image of a starry sky that evokes the image of the song "Twinkle, Twinkle, Little Star" which is the song planned to be sung next.

Next, the nursing care robot 11 begins to introduce and explain the song ("Twinkle, Twinkle, Little Star") that is about to be sung by executing a speech motion that includes the following lines.

"The stars are beautiful in the winter sky, aren't they?"

"Shall we sing the song about the stars, Twinkle, Twinkle, Little Star?"

Next, the nursing care robot 11 has the image in Fig. 30 at A displayed on the hand display 91. The image in Fig. 30 at A shows the title of the song "Twinkle, Twinkle, Little Star" that is about to be sung.

Next, the nursing care robot 11 executes a speech motion that includes the following lines, finishes the introduction and description of the song ("Twinkle, Twinkle, Little Star") that is about to be sung, and completes the preparation for care.

"Twinkle, Twinkle, Little Star."

"It's Twinkle, Twinkle, Little Star."

Next, the nursing care robot 11 executes perception connection by singing "Twinkle, Twinkle, Little Star" with the care recipient 14, while having the hand display 91 display an image showing the lyrics of "Twinkle, Twinkle, Little Star" as shown in Fig. 30 at B.

After singing "Twinkle, Twinkle, Little Star," the nursing care robot 11 has the hand display 91 display an image showing "Thank you" as shown in Fig. 30 at C. The nursing care robot 11 then begins emotional fixation by executing a speech motion that includes the following lines.

"Thank you."

Next, the nursing care robot 11 executes a speech motion that includes the following lines.

"Grandma, you look very nice, thank you."

"I'm glad you are laughing."

Next, the nursing care robot 11 executes a speech motion that includes the following lines to end the emotional fixation.

"I am glad."

Next, the nursing care robot 11 has the hand display 91 display an image related to the "Rudolph the Red-Nosed Reindeer," (not shown) which is planned to be sung next. The nursing care robot 11 then executes a speech motion that includes the following lines and outputs a bell sound, thereby performing cognitive stimulation therapy and initiating the preparation for care.

"Next."

"Quiz, quiz."

"What is this sound?"

Next, the nursing care robot 11 executes a speech motion that includes the following lines if the care recipient 14 answers the quiz correctly.

"Yes, you are correct."

"This is the sound of the reindeer's bells."

Next, the nursing care robot 11 begins to introduce and explain about the song ("Rudolph the Red-Nosed Reindeer") that is about to be sung by executing a speech motion that includes the following lines.

"Shall we sing the Christmas song, "Rudolph the Red-Nosed Reindeer?""

Next, the nursing care robot 11 executes a speech motion that includes the following lines, and completes the introduction and explanation of the song ("Rudolph the Red-Nosed Reindeer") that is about to be sung, thereby completing the preparation for care.

"Rudolph the Red-Nosed Reindeer."

"It's Rudolph the Red-Nosed Reindeer."

Next, the nursing care robot 11 performs perception connection by singing "Rudolph the Red-Nosed Reindeer" with the care recipient 14 while having the hand display 91 display an image showing the lyrics of "Rudolph the Red-Nosed Reindeer" (not shown).

After singing "Rudolph the Red-Nosed Reindeer," the nursing care robot 11 has the hand display 91 display the image showing "Thank you" in Fig. 30 at C. The nursing care robot 11 then begins emotional fixation by executing a speech motion that includes the following line.

"Thank you."

Next, the nursing care robot 11 executes a speech motion that includes the following lines.

"Grandma."

"It was so nice of you to sing with me."

Next, the nursing care robot 11 executes a speech motion that includes the following lines.

"Very nice."

"Sing with me again."

"Look forward to it."

Next, the nursing care robot 11 executes a speech motion that includes the following line.

"I'm so glad."

Next, the nursing care robot 11 executes a speech motion that includes the following lines and ends the emotional fixation.

"This concludes our singing time."

"It was fun singing today, wasn't it?"

"Thank you."

Next, the nursing care robot 11 carries out the promise to meet again by executing a speech motion that includes the following lines.

"It was nice to meet you today."

"I will come to see you again."

"See you."

The nursing care robot 11 then ends the music therapy application and moves away from the care recipient 14.

In this way, the nursing care robot 11 can perform the music therapy while increasing the receptiveness of the care recipient 14 to the nursing care robot 11 and the song using Humanitude.

The scenario and content of the music therapy application (hereinafter referred to as "scenario, etc.") may vary depending on temporal factors such as seasons and months. For example, the images presented to the care recipient, the lines spoken by the nursing care robot 11, and the songs sung with the care recipient may change seasonally or monthly.

For example, multiple scenarios, etc. are prepared in advance for each month, and one of them is executed. The scenario, etc. to be executed may be randomly selected according to the wishes of the care recipient, selected by the staff for each care recipient, or switched on a weekly basis.

For example, on special days such as birthdays and anniversaries, special scenarios can be executed. For example, a scenario, etc. that include a birthday song or congratulatory message may be executed on the care recipient's birthday.

### <Telephone application>

Next, for example with reference to Figs. 31 to 36, a specific example of a telephone application in which the care recipient 14 converses with a caller via a video phone will be described.

First, the nursing care robot 11 looks at the hand display 91 at the initial position P0, as shown in Fig. 31, then moves to the long-distance position P3, as shown in Fig. 32, and executes the preparation for meeting by performing a speech motion that includes the following lines.

"Grandma!"

"Good morning!"

"Grandma! I'm coming to you now."

In response to this, if the care recipient 14 does not gaze at the nursing care robot 11, the nursing care robot 11 repeats this speech motion. If the care recipient 14 still does not gaze at the nursing care robot 11, the nursing care robot 11 ends the telephone application and moves away from the care recipient 14.

Meanwhile, if the care recipient 14 gazes at the nursing care robot 11, the nursing care robot 11 moves to the medium-distance position P2 as shown in Fig. 25 and begins the preparation for care by performing a speech motion that includes the following lines. At the time, the nursing care robot 11 performs cognitive stimulation therapy and executes the introduction of the call by displaying the face image of the caller on the hand display 91 and showing it to the care recipient 14.

"I am glad you are laughing, Grandma."

"You look nice today as always."

"Now with Grandma."

"On the phone."

"There's someone who wants to talk to you."

"That's so sweet."

"Guess who?"

Next, the nursing care robot 11 executes a speech motion that includes the following lines.

"I'm going to call that person now."

"Just wait for a moment, please."

Next, nursing care robot 11 makes a video call to the caller of the care recipient 14 using the hand display 91. When the caller answers the video call, the nursing care robot 11 executes a speech motion that includes the following lines.

"Hello, this is Hanamo speaking."

"Thank you for answering my call."

"Grandma, it's time to talk over the phone."

"I'll put Grandma on the phone."

Next, the nursing care robot 11 moves to the short-distance position P1, executes a speech motion that includes the following line, places the hand display 91 on the table 12 as shown in Fig. 33, and completes the preparation for care.

"I'll put the phone on the table."

The care recipient 14 then experiences the perception connection by using the hand display 91 to talk to the care recipient 14.

Next, the nursing care robot 11 executes a speech motion that includes the following lines at the appropriate timing, ends the video call, and initiates the emotional fixation.

"It's almost time for singing."

"Thank you for talking on the phone."

"Let's talk on the phone again."

Next, the nursing care robot 11 executes a speech motion that includes the following lines.

"Let's make a phone call again sometime."

"Let's do it again."

"Let's do it again!"

Next, the nursing care robot 11 lowers the hand 63 and moves the hand display 91 off of the table 12.

"I'm going to lower my hand now."

After lowering the hand 63, the nursing care robot 11 executes a speech motion that includes the following line.

"I have lowered my hand."

Next, the nursing care robot 11 executes a speech motion that includes the following lines.

"This concludes the time for telephone call."

"It was nice to talk, wasn't it?"

"Thank you."

Next, the nursing care robot 11 executes a speech motion that includes the following lines.

"I'm glad you are laughing."

"I see you love everyone."

"Thank you for letting me know about it."

"Let's make a phone call again sometime."

Next, the nursing care robot 11 executes a speech motion that includes the following line to end the emotional fixation.

"Thank you."

Next, after moving to the medium-distance position P2 as shown in Fig. 34, the nursing care robot 11 executes a speech motion that includes the following lines to start the promise to meet again.

"Next, it's time for singing."

"I'm going to get ready for singing."

"See you."

Next, the nursing care robot 11 executes a speech motion that includes the following lines to end the promise to meet again.

"I'll be back."

"See you."

Next, the nursing care robot 11 moves to the long-distance position P3 and stops as shown in Fig. 35. The nursing care robot 11 then gazes at the care recipient 14 as shown in Fig. 36, then ends the telephone application and moves away from the care recipient 14.

In the telephone application, the video telephone does not have to be used, and a voice-only phone may be used. In this case, for example, a picture of the caller's face is displayed on the hand display 91. Depending on the wishes of the caller or other factors, an image other than the face photo of the caller may be displayed on the hand display 91.

In the telephone application, t is not always necessary to execute a video call in real-time, and for example, a message video produced in advance can be played using a video playback application.

For example, if the care recipient's family, friends, or other caller is unable to make a call in real time, a message video produced in advance by the caller may be reproduced.

In this case, for example, the caller uploads the produced video message to the cloud server, and the nursing care robot 11 downloads the video message from the cloud server and plays it. For example, the information terminal at the nursing care facility may download the video message from the cloud server and send it to the nursing care robot 11.

For example, when the caller comes to the nursing care facility, the nursing care robot 11 may take a picture of the caller, produce a video message, and store it.

The speaker in the message video does not have to be a real-life caller of the care recipient. For example, the speaker could be a celebrity such as an entertainer or an athlete, a character from movies, TV shows, or manga, or a character generated by computer graphics or animation.

The speaker in the message video may be for example a staff member of the nursing care facility. This is expected to improve the relationship between the staff member and the care recipient.

Furthermore, instead of the video message, an sound message may be used. In this case, for example, a picture of the speaker is displayed on the hand display 91.

### <Standing and sitting detection processing>

Next, standing and sitting detection processing performed by the nursing care robot 11 will be described with reference to the flowchart in Fig. 37.

This processing is performed, for example, when the nursing care robot 11 is at a prescribed location in the living area for charging (where the charging dock 212 (not shown) is located) as shown in Figs. 38 and 39, and a watch over application that watches over the users in the living area is in effect.

Tables 401-1 to 401-8 and chairs 402-1 to 402-7 are located in the living area.

The tables 401-1 to 401-8 are arranged in a U-shape. In other words, the row of tables 401-1 and 401-2, the row of tables 401-3 to 401-6, and the row of tables 401-7 and 401-8 are arranged in a U-shape. The row of tables 401-3 to 401-6 is arranged in front of the nursing care robot 11. The row of tables 401-1 and 401-2 and the row of tables 401-7 and 401-8 are arranged perpendicular to the row of tables 401-3 to 401-6 and opposed to each other.

Chairs 402-1 to 402-7 are arranged for the tables 401-1 to 401-7, respectively. For table 401-8, no chair is arranged.

In Figs. 38 and 39, the long-distance position P3, the medium-distance position P2, and the short-distance position P1 are shown for each of the chairs 402-1 to 402-7.

In step S301, the nursing care robot 11 looks over the living area. For example, the motor drive control unit 326 drives the actuator 239 to turn the neck joint axis 24C under the control of the scenario execution control unit 324. In this way, the head 21 of the nursing care robot 11 swings from side to side, and the distance image sensor 231 takes an image of the area that includes the tables 401 and the chairs 402 in the living area. The distance image sensor 231 provides the distance image data obtained from the imaging to the image information processing unit 281.

When the tables 401 and the chairs 402 are within the field of vision of the distance image sensor 231, the nursing care robot 11 does not have to swing its head 21 from side to side.

In step S302, the nursing care robot 11 checks the presence of each user. Specifically, the image information processing unit 281 checks the presence of each user by detecting whether the user is seated in any of the chairs 402 on the basis of the distance image data. The image information processing unit 281 supplies information indicating the result of the presence check to the action control unit 264.

In step S303, the image information processing unit 281 determines whether any of the users has stood up. On the basis of the result of the processing in step S302, upon determining that at least one of the users who are present has stood up, the image information processing unit 281 determines that the user's act of standing up has been detected, and the process proceeds to step S304.

For example, if it is determined that a user sitting in a wheelchair without using the chair 402 has stood up, the detection of the user's act of standing up is determined in the same manner.

In step S304, the nursing care robot 11 notifies the staff of the user's act of standing up. For example, the call communication function control unit 330 transmits information via a Wi-Fi router 213 and a facility LAN 214 to an external device (e.g., an information terminal (e.g., a smartphone) carried by a staff member or an information terminal in the staff room) to notify the user's act of standing up.

In response to this, the external device notifies the staff of the user's act of standing up by at least one of visual, auditory, and tactile information, for example, on the basis of the received information.

In step S305, the nursing care robot 11 records the user's standing up history. For example, the call communication function control unit 330 transmits data indicating the result of detecting the user's act of standing up via the Wi-Fi router 213 and the facility LAN 214 to an information terminal that runs care recording software that records the history of care for each user.

In response, the information terminal records the user's standing up history in the care record software according to the received data.

After that, the process returns to step S301, and the processing from step S301 onward is executed.

Meanwhile, if it is determined in step S303 that a user's act of standing up is not detected, the process returns to steps S301, and the processing in step S301 onward is executed.

This allows the staff to be quickly aware of the user's act of standing up and take the necessary measures. As a result, this helps prevent incidents such as the user's injuries or wandering by the user.

For example, before executing each application according to the schedule information, the nursing care robot 11 can be aware in advance of whether the care recipient for whom each application is to be executed is present or not. The nursing care robot 11 can then skip executing applications for care recipients who are not present, thus enabling smoother execution of each application.

If, for example, the nursing care robot 11 is unable to take images of all seated users from only one location in the living area because of the blind spots of the nursing care robot 11, the nursing care robot 11 may move between multiple locations while executing the application for watching over the care recipient.

In this case, for example, multiple standby positions are provided in the living area, and the nursing care robot 11 periodically travels between the standby positions to watch over the users in the living area by taking images in the living area while moving and from each standby position. Each standby position is provided with a charging dock 212, if necessary.

This reduces blind spots and ensures that users within the living area are watched over from appropriate positions, regardless of the shape or layout of the living area. When compared to installing surveillance cameras in the living area, more flexibility is allowed in adapting to changes in the layout of the living area and other changes.

For example, the processing in steps S303 to S305 is also performed while the nursing care robot 11 executes the application. When an act of standing up by a user is detected, the staff is notified and the user's standing up history is recorded.

### <Vital measurement processing>

Next, with reference to the flowchart in Fig. 40, the vital measurement processing performed by the nursing care robot 11 will be described.

The processing is performed by the nursing care robot 11 to each care recipient for example when a vital measurement application is executed.

In step S351, the nursing care robot 11 executes vital measurement. For example, the motor drive control unit 326, under the control of the scenario execution control unit 324, drives the actuator 239 to move the nursing care robot 11 to measure the value of the vital signs of a care recipient using the vital sensor 233. The vital sensor 233 supplies the vital data indicating the measurement result of the value of the vital signs of the care recipient to the vital information processing unit 283. The vital information processing unit 283 executes the detection processing of the value of the vital signs of the care recipient on the basis of the vital data. The vital information processing unit 283 supplies the vital information indicating the detection result of the value of the vital signs to the action control unit 264.

In step S352, the scenario execution control unit 324 determines whether a measurement error has occurred. If at least one of the value of the vital signs failed to be measured, or if at least one of the value of the vital signs is an error value, the scenario execution control unit 324 determines that a measurement error has occurred, and the process proceeds to step S353. For example, if one of the value of the vital signs, the measured temperature of the nursing caregiver, is an impossible value (e.g., at least 41°C), it is determined that a measurement error has occurred.

In step S353, the scenario execution control unit 324 determines whether a measurement error has occurred consecutively at least a prescribed number of times (e.g., four times). If it is determined that the number of consecutive measurement errors has not reached the prescribed number, the process returns to step S351.

Thereafter, until it is determined in step S352 that no measurement error has occurred or it is determined in step S353 that the number of consecutive measurement errors has reached the prescribed number, the processing in steps S351 to S353 is repeatedly performed.

Meanwhile, if it is determined in step S352 that no measurement error has occurred, the process proceeds to step S354.

In step S354, the scenario execution control unit 324 determines whether any of the value of the vital signs is abnormal. Specifically, the scenario execution control unit 324 determines that the value of the vital signs are abnormal if at least one of the value of the vital signs of the care recipient is within an abnormal range, and the process proceeds to step S355. For example, if one of the value of the vital signs, the body temperature of the care recipient, is at least 37.5°C and less than 40°C, the value of the vital signs are determined to be abnormal.

The normal and abnormal ranges of each the value of the vital sign may be set for each care recipient.

In step S355, the nursing care robot 11 notifies the staff of the abnormal value of the vital sign. For example, the call communication function control unit 330 transmits information via the Wi-Fi router 213 and the facility LAN 214 to an external device (e.g., an information terminal (e.g., a smartphone) carried by a staff member of the nursing care facility or an information terminal in the staff room) to notify the abnormal value of the vital sign of the care recipient.

In response to this, the external device, for example, notifies the staff of abnormality in the value of the vital sign of the care recipient by at least one of visual, auditory, and tactile information on the basis of the received information.

Thereafter, the process proceeds to step S356.

Meanwhile, in step S354, if the value of the vital signs of the care recipient are all within the normal range, the scenario execution control unit 324 determines that the value of the vital signs are normal, the processing in step S355 is skipped and the process proceeds to step S356.

When it is determined in step S353 that a measurement error has occurred consecutively at least a prescribed number of times, the processing in steps S354 and S355 is skipped, and the process proceeds to step S356.

In step S356, the nursing care robot 11 records the value of the vital signs of the care recipient. For example, the call communication function control unit 330 transmits the vital information indicating the value of the vital signs of the care recipient via the Wi-Fi router 213 and the facility LAN 214 to the information terminal that runs the care recording software.

In response to this, the information terminal records the received vital information about the care recipient in the care recording software. If a measurement error occurs, the measurement error occurrence is recorded in the nursing care record software.

Thereafter, the process returns to step S351, and the processing from step S351 onward is executed.

This allows the staff to be quickly aware of any abnormality in the value of the vital signs of the care recipient and take the necessary measures. As a result, appropriate measures can be promptly taken for example in response to a poor physical condition of the care recipient.

As described above, using Humanitude, the nursing care robot 11 can have the care recipient 14 talk on the video phone while increasing the receptivity of the care recipient 14 to the nursing care robot 11 and the telephone.

As described above, according to the present technology, the receptivity of the care recipient to care by the nursing care robot 11 may be improved. For example, the recognition and receptivity of the care recipient to the provided content may be improved. For example, in music therapy, it becomes easier for the care recipient to be aware in advance of what song is to be sung, which makes it easier for them to sing the song. As a result, high quality care suitable for each care recipient can be provided.

This allows the care recipient to lead a calm life in the absence of staff or in his/her own room. The quality of life of the care recipient is improved. The rhythm of the care recipient's life can be regulated, and the instability of the care recipient can be prevented. If the care recipient is a patient with dementia, non-pharmacological therapy can be carried out to the care recipient on a regular basis using the nursing care robot 11, which is expected to have the effect of slowing the progression of dementia.

In addition, staff members may carry out their care duties with a sense of security, and the entire care operation may become more efficient, leading to cost reductions.

### <<2. Modifications>>

Hereinafter, modifications of the foregoing embodiments of the present technology will be described.

In the examples described above, the nursing care robot 11 operates autonomously, but the nursing care robot 11 may be operated by remote control by an external information processing device such as a mobile information terminal or a cloud server. In this case, all or some of the functions of the robot internal PC 235 (e.g., the action control unit 264) are provided in an external device.

The shape, specifications, etc. of the nursing care robot may be changed as appropriate according to the content and purpose of care.

The path by which the nursing care robot 11 approaches the care recipient 14 is not limited to the example shown in Fig. 19, and can be modified as required. For example, the nursing care robot 11 may be configured to stop at four or more locations.

### <<3. Others>>

### <Exemplary computer configuration>

The series of processing described above can be executed by hardware or software. When the series of processing is performed by software, the program constituting the software is installed on the computer. Here, the computer includes a computer embedded in dedicated hardware or, for example, a general-purpose personal computer capable of executing various functions by installing various programs.

Fig. 41 is a block diagram showing an example of a hardware configuration of a computer that executes the above-described series of processing according to a program.

In a computer 1000, a central processing unit (CPU) 1001, a read only memory (ROM) 1002, and a random access memory (RAM) 1003 are connected to each other by a bus 1004.

An input/output interface 1005 is further connected to the bus 1004. An input unit 1006, an output unit 1007, a storage unit 1008, a communication unit 1009, and a drive 1010 are connected to the input/output interface 1005.

The input unit 1006 includes for example an input switch, a button, a microphone, and an imaging element. The output unit 1007 includes for example a display and a speaker. The storage unit 1008 includes for example a hard disk and a non-volatile memory. The communication unit 1009 may be a network interface. The drive 1010 drives a removable medium 1011 such as a magnetic disk, an optical disc, a magneto-optical disk, or a semiconductor memory.

In the computer 1000 configured as described above, for example, the CPU 1001 loads a program recorded in the storage unit 1008 into the RAM 1003 via the input/output interface 1005 and the bus 1004 and executes the program to perform the series of processing described above.

The program executed by the computer 1000 (CPU 1001) may be recorded on, for example, the removable medium 1011 for example as a package medium so as to be provided. The program can also be provided via a wired or wireless transmission medium such as a local area network, the Internet, or digital satellite broadcasting.

In the computer 1000, the program may be installed in the storage unit 1008 via the input/output interface 1005 by inserting the removable medium 1011 into the drive 1010. Further, the program can be received by the communication unit 1009 via the wired or wireless transmission medium and installed in the storage unit 1008. Alternatively, the program can be installed in the ROM 1002 or the storage unit 1008 in advance.

Note that the program executed by a computer may be a program that performs processing chronologically in the order described in the present specification or may be a program that performs processing in parallel or at a necessary timing such as a called time.

In the present specification, a system means a set of a plurality of constituent elements (devices, modules (components), or the like) and all the constituent elements may or may not be included in a same casing. Accordingly, a plurality of devices accommodated in separate casings and connected via a network and one device in which a plurality of modules are accommodated in one casing both constitute systems.

Further, embodiments of the present technique are not limited to the above-mentioned embodiment and various modifications may be made without departing from the gist of the present technique.

For example, the present technique may be configured as cloud computing in which a plurality of devices share and cooperatively process one function via a network.

In addition, each step described in the above flowchart can be executed by one device or executed in a shared manner by a plurality of devices.

Furthermore, in a case in which one step includes a plurality of processes, the plurality of processes included in the one step can be executed by one device or executed in a shared manner by a plurality of devices.

### <Combination example of configuration>

The present technology can also have the following configuration.

(1)
   A nursing care robot including an action control unit configured to execute a cognitive approach that stepwise reduces a cognitive distance of a care recipient to content provided in an application for providing care to the care recipient and then to provide the content.
(2)
   The nursing care robot according to (1), wherein the action control unit executes a physical approach that stepwise reduces a physical distance to the care recipient before executing the cognitive approach.
(3)
   The nursing care robot according to (2), wherein the action control unit has the nursing care robot approach the care recipient stepwise while executing, in each of multiple positions at different distances, at least one speech motion including at least one of speech and a motion.
(4)
   The nursing care robot according to (3), wherein the action control unit executes the at least one speech motion in a first position and then move the nursing care robot to a second position closer to the care recipient than the first position.
(5)
   The nursing care robot according to (4), further including a sensor information processing unit configured to detect a gaze from the care recipient at the nursing care robot according to information from a sensor,
   wherein
   the action control unit executes a first speech motion and then controls execution of the next second speech motion according to the presence or absence of the gaze from the care recipient at the nursing care robot.
(6)
   The nursing care robot according to (5), wherein after executing the first speech motion, if the care recipient gazes at the nursing care robot, the action control unit executes the second speech motion.
(7)
   The nursing care robot according to (5) or (6), wherein after executing the first speech motion, if the care recipient speaks, the action control unit executes a response motion including a response to the speech by the care recipient before executing the second speech motion.
(8)
   The nursing care robot according to (7), wherein after executing the first speech motion, if the care recipient speaks while gazing at the nursing care robot, the action control unit executes the response motion before executing the second speech motion.
(9)
   The nursing care robot according to (7) or (8), wherein the action control unit selects the response to be executed according to a phrase included in the speech by the care recipient.
(10)
   The nursing care robot according to any one of (5) to (9), wherein after executing the first speech motion, if the care recipient does not gaze at the nursing care robot, the action control unit executes at least a part of the first speech motion again.
(11)
   The nursing care robot according to (10), wherein after executing the first speech motion, if the care recipient does not gaze at the nursing care robot, the action control unit executes an additional motion for encouraging the care recipient to gaze and at least a part of the first speech motion again.
(12)
   The nursing care robot according to (10) or (11), wherein after executing the first speech motion, if the care recipient does not gaze at the nursing care robot, the action control unit executes at least a part of the first speech motion again with different sound.
(13)
   The nursing care robot according to any one of (10) to (12), wherein the action control unit ends the application if the care recipient does not gaze at the nursing care robot for at least a prescribed period.
(14)
   The nursing care robot according to any one of (1) to (13), wherein the action control unit guides the care recipient to the content by presenting information related to the content.
(15)
   The nursing care robot according to (14), wherein the action control unit presents information related to the content by at least one of conveying orientation information related to the content, asking a question related to the content, and conveying information that directly represents the content.
(16)
   The nursing care robot according to (14) or (15), wherein the action control unit guides the care recipient to the content by at least one of reality orientation, cognitive stimulation therapy, introduction of the content, and explanation of the content.
(17)
   The nursing care robot according to any one of (1) to (16), wherein upon detecting an act of standing up from a seat by the care recipient, the action control unit notifies an external device of the act of standing up of the care recipient.
(18)
   The nursing care robot according to any one of (1) to (17), wherein upon detecting an abnormality in a value of a vital sign of the care recipient while executing the application for measuring the value of the vital sign of the care recipient, the action control unit notifies an external device of the abnormality in the value of the vital sign of the care recipient.
(19)
   A method for controlling a nursing care robot configured to execute an application for providing care to a care recipient, the method including causing the nursing care robot to execute a cognitive approach that stepwise reduces a cognitive distance of the care recipient to content to be provided in the application and then to provide the content.
(20)
   An information processing device including an action control unit configured to cause a nursing care robot, which executes an application for providing care to a care recipient, to execute a cognitive approach that stepwise reduces a cognitive distance of the care recipient to content to be provided in the application and then to provide the content.

The advantageous effects described in the present specification are merely exemplary and are not limited, and other advantageous effects may be obtained.

### [Reference Signs List]

- 11: Nursing care robot
- 14: Care recipient
- 21: Head
- 63L, 63R: Hand
- 71: Cart
- 81: Head sensor
- 82: Chest sensor
- 83L, 83R: Hand sensor
- 91: Hand display
- 113F: Front wheel
- 113B: Rear wheel
- 113L: Left wheel
- 113R: Right wheel
- 114F to 114R: Damper
- 115F to 115R: Spring
- 201: Robot operation system
- 231: Distance image sensor
- 232: Microphone
- 233: Vital sensor
- 234: LiDAR
- 235: Robot internal PC
- 238: Speaker
- 239: Actuator
- 242: Cart drive unit
- 243: Actuator
- 261: Sensor Information management unit
- 263: Application control unit
- 264: Action control unit
- 281: Image information processing unit
- 282: Sound information processing unit
- 324: Scenario execution control unit
- 325: Tracking motion control unit
- 326: Motor drive control unit
- 327: Sound output control unit
- 328: Navigation control unit
- 330: Call communication function control unit

## Claims

1. A nursing care robot comprising an action control unit configured to execute a cognitive approach that stepwise reduces a cognitive distance of a care recipient to content provided in an application for providing care to the care recipient and then to provide the content.

2. The nursing care robot according to claim 1, wherein the action control unit executes a physical approach that stepwise reduces a physical distance to the care recipient before executing the cognitive approach.

3. The nursing care robot according to claim 2, wherein the action control unit has the nursing care robot approach the care recipient stepwise while executing, in each of multiple positions at different distances, at least one speech motion including at least one of speech and a motion.

4. The nursing care robot according to claim 3, wherein the action control unit executes the at least one speech motion in a first position and then move the nursing care robot to a second position closer to the care recipient than the first position.

5. The nursing care robot according to claim 4, further comprising a sensor information processing unit configured to detect a gaze from the care recipient at the nursing care robot according to information from a sensor,
wherein
the action control unit executes a first speech motion and then controls execution of the next second speech motion according to the presence or absence of the gaze from the care recipient at the nursing care robot.

6. The nursing care robot according to claim 5, wherein after executing the first speech motion, if the care recipient gazes at the nursing care robot, the action control unit executes the second speech motion.

7. The nursing care robot according to claim 5, wherein after executing the first speech motion, if the care recipient speaks, the action control unit executes a response motion including a response to the speech by the care recipient before executing the second speech motion.

8. The nursing care robot according to claim 7, wherein after executing the first speech motion, if the care recipient speaks while gazing at the nursing care robot, the action control unit executes the response motion before executing the second speech motion.

9. The nursing care robot according to claim 7, wherein the action control unit selects the response to be executed according to a phrase included in the speech by the care recipient.

10. The nursing care robot according to claim 5, wherein after executing the first speech motion, if the care recipient does not gaze at the nursing care robot, the action control unit executes at least a part of the first speech motion again.

11. The nursing care robot according to claim 10, wherein after executing the first speech motion, if the care recipient does not gaze at the nursing care robot, the action control unit executes an additional motion for encouraging the care recipient to gaze and at least a part of the first speech motion again.

12. The nursing care robot according to claim 10, wherein after executing the first speech motion, if the care recipient does not gaze at the nursing care robot, the action control unit executes at least a part of the first speech motion again with different sound.

13. The nursing care robot according to claim 10, wherein the action control unit ends the application if the care recipient does not gaze at the nursing care robot for at least a prescribed period.

14. The nursing care robot according to claim 1, wherein before providing the content, the action control unit guides the care recipient to the content by presenting information related to the content.

15. The nursing care robot according to claim 14, wherein the action control unit presents information related to the content by at least one of conveying orientation information related to the content, asking a question related to the content, and conveying information that directly represents the content.

16. The nursing care robot according to claim 14, wherein the action control unit guides the care recipient to the content by at least one of reality orientation, cognitive stimulation therapy, introduction of the content, and explanation of the content.

17. The nursing care robot according to claim 1, wherein upon detecting an act of standing up from a seat by the care recipient, the action control unit notifies an external device of the act of standing up of the care recipient.

18. The nursing care robot according to claim 1, wherein upon detecting an abnormality in a value of a vital sign of the care recipient while executing the application for measuring the value of the vital sign of the care recipient, the action control unit notifies an external device of the abnormality in the value of the vital sign of the care recipient.

19. A method for controlling a nursing care robot configured to execute an application for providing care to a care recipient, the method comprising causing the nursing care robot to execute a cognitive approach that stepwise reduces a cognitive distance of the care recipient to content to be provided in the application and then to provide the content.

20. An information processing device comprising an action control unit configured to cause a nursing care robot, which executes an application for providing care to a care recipient, to execute a cognitive approach that stepwise reduces a cognitive distance of the care recipient to content to be provided in the application and then to provide the content.
